(19) Europäisches Patentamt
European Patent Office
Office européen des brevets

(11) Veröffentlichungsnummer: **0 303 114 B1**

# (12) EUROPÄISCHE PATENTSCHRIFT

(45) Veröffentlichungstag der Patentschrift: **24.08.94**

(51) Int. Cl.5: **C07D 239/42**, C07D 239/47, C07D 239/52, C07D 239/48, C07D 239/56, C07D 251/44, C07D 251/46, C07D 251/48, C07D 251/52, C07D 249/14, A01N 47/36

(21) Anmeldenummer: **88112280.8**

(22) Anmeldetag: **29.07.88**

Die Akte enthält technische Angaben, die nach dem Eingang der Anmeldung eingereicht wurden und die nicht in dieser Patentschrift enthalten sind.

## (54) Heterocyclisch substituierte Sulfamidsäurephenylester, Verfahren zu ihrer Herstellung und ihre Verwendung als Herbizide und Pflanzenwachstumsregulatoren.

(30) Priorität: **05.08.87 DE 3725939**
**27.02.88 DE 3806323**

(43) Veröffentlichungstag der Anmeldung:
**15.02.89 Patentblatt 89/07**

(45) Bekanntmachung des Hinweises auf die Patenterteilung:
**24.08.94 Patentblatt 94/34**

(84) Benannte Vertragsstaaten:
**BE CH DE ES FR GB IT LI NL**

(56) Entgegenhaltungen:
**EP-A- 0 004 163**
**EP-A- 0 030 138**
**EP-A- 0 098 569**
**EP-A- 0 135 332**
**DE-A- 3 105 453**
**DE-A- 3 151 450**

**PATENT ABSTRACTS OF JAPAN Band 7, Nr. 6 (C-161)(1231),9. April 1983; & JP - A - 5815962 (MITSUI TOATSU KAGAKU K.K.) 29.01.1983**

(73) Patentinhaber: **Hoechst Schering AgrEvo GmbH**
**Gerichtstrasse 27**
**D-13342 Berlin (DE)**

(72) Erfinder: **Willms, Lothar, Dr.**
**Schulstrasse 3**
**D-5411 Hillscheid (DE)**
Erfinder: **Kehne, Heinz, Dr.**
**Berliner Strasse 10**
**D-6238 Hofheim am Taunus (DE)**
Erfinder: **Bauer, Klaus, Dr.**
**Doorner Strasse 53D**
**D-6450 Hanau (DE)**
Erfinder: **Bieringer, Hermann, Dr.**
**Eichenweg 26**
**D-6239 Eppstein/Taunus (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Es ist bekannt, daß heterocyclisch substituierte Phenoxysulfonylharnstoffe herbizide und pflanzenwachstumsregulierende Eigenschaften aufweisen (EP-A-4163, EP-A-165572, EP-A-0135332), von denen einige in 2-Stellung im Phenoxyrest eine Alkoxycarbonylgruppe aufweisen. Aus EP-A-030138 sind außerdem analog strukturierte Phenylsulfonylharnstoffe bekannt, die in 2-Stellung am Phenylring unter anderem eine substituierte Alkoxycarbonylgruppe aufweisen.

Die bekannten Phenoxysulfonylharnstoffherbizide weisen bei ihrer Anwendung Nachteile auf, wie beispielsweise eine hohe Persistenz oder unzureichende Selektivität.

Es wurde nun gefunden, daß heterocyclisch substituierte Sulfamidsäurephenylester, die im Phenylesterteil spezielle Alkoxycarbonylgruppen enthalten, als Herbizide und Pflanzenwachstumsregulatoren besonders geeignet sind.

Gegenstand der vorliegenden Erfindung sind daher Verbindungen der Formel (I) oder deren Salze

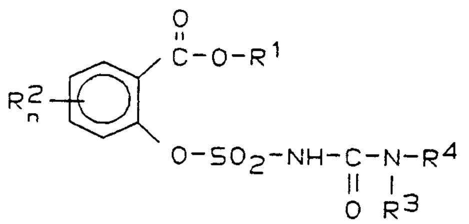

(I),

worin

| | |
|---|---|
| $R^1$ | $(C_1$-$C_8)$-Alkyl, das ein- oder mehrfach durch Halogen oder ein- bis zweifach durch $(C_1$-$C_4)$-Alkoxy substituiert ist; $(C_2$-$C_8)$-Alkenyl, $(C_2$-$C_8)$-Alkinyl, die beide gegebenenfalls ein- oder mehrfach durch Halogen oder ein- bis zweifach durch $(C_1$-$C_4)$-Alkoxy substituiert sein können, |
| $R^2$ | unabhängig voneinander Halogen; Nitro; $(C_1$-$C_4)$-Alkyl oder $(C_1$-$C_4)$-Alkoxy, die beide gegebenenfalls ein- oder mehrfach durch Halogen substituiert sein können, |
| $R^3$ | Wasserstoff; $(C_1$-$C_8)$-Alkyl; $(C_2$-$C_8)$-Alkenyl oder $(C_2$-$C_8)$-Alkinyl, |
| $R^4$ | einen heterocyclischen Rest der Formeln |

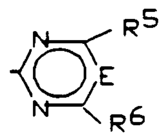

oder

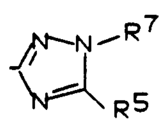

,

| | |
|---|---|
| | wobei E = CH oder N ist, |
| n | 0, 1, 2 oder 3, |
| $R^5$,$R^6$ | unabhängig voneinander Wasserstoff; Halogen; $(C_1$-$C_4)$-Alkyl oder $(C_1$-$C_4)$-Alkoxy, die beide gegebenenfalls ein- oder mehrfach halogeniert sein können; Di-$(C_1$-$C_4)$-alkoxy-$(C_1$-$C_2)$-alkyl; Cyclopropyl, $-OCHR^8COOR^9$; $-NR^9R^{10}$ oder $(C_1$-$C_4)$-Alkylthio, |
| $R^7$ | $(C_1$-$C_4)$-Alkyl, |
| $R^8$ | Wasserstoff oder $(C_1$-$C_4)$-Alkyl und |
| $R^9$,$R^{10}$ | unabhängig voneinander Wasserstoff; $(C_1$-$C_4)$-Alkyl; $(C_2$-$C_4)$-Alkenyl oder $(C_2$-$C_4)$-Alkinyl |

bedeuten.

Die Verbindungen der Formel I können Salze bilden, bei denen der Wasserstoff der $-SO_2$-NH-Gruppe durch ein für die Landwirtschaft geeignetes Kation ersetzt wird. Diese Salze sind im allgemeinen Metall-, insbesondere Alkali-, Erdalkali-, gegebenenfalls alkylierte Ammonium- oder organische Aminsalze. Sie werden vorzugsweise in inerten Lösungsmitteln wie z. B. Wasser, Methanol oder Aceton bei Temperaturen von 0 - 100 °C hergestellt. Geeignete Basen zur Herstellung der erfindungsgemäßen Salze sind beispielsweise Alkalicarbonate, wie Kaliumcarbonat, Alkali- und Erdalkalihydroxide, Ammoniak oder Ethanolamin.

Bevorzugte Verbindungen der Formel I sind solche, bei denen $R^1$ $(C_1$-$C_4)$-Alkyl, das wie oben beschrieben substituiert ist, oder $(C_3$-$C_4)$-Alkenyl, das wie oben beschrieben substituiert sein kann oder Propargyl; $R^2$ Halogen, $(C_1$-$C_3)$-Alkyl oder $(C_1$-$C_3)$-Alkoxy, die wie oben beschrieben substituiert sein können, wobei $R^2$ bevorzugt in Position 3 (bezogen auf den Rest $COOR^1$) des Phenylrings orientiert ist; n = 0, 1 oder 2; $R^3$ Wasserstoff, $(C_1$-$C_4)$-Alkyl oder Allyl; $R^4$ einen Rest der Formel

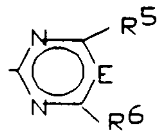

und $R^5$ und $R^6$ unabhängig voneinander Halogen, $(C_1\text{-}C_4)$-Alkyl oder $(C_1\text{-}C_4)$-Alkoxy, die beide durch Halogen substituiert sein können, bedeuten, sowie deren Salze.

Halogen bedeutet vorzugsweise Fluor, Chlor oder Brom. Unter halogeniertem Alkyl oder halogeniertem Alkoxy sind insbesondere zu verstehen die Reste $CF_3$, $CH_2\text{-}CH_2Cl$, $CH_2CH_2Br$, $CH_2CF_3$, $OCF_2H$, $OCH_2CF_3$ .

Halogeniertes Alkenyl oder halogeniertes Alkinyl bedeutet insbesondere $CH_2CH=CHCl$, $CH_2CCl=CCl_2$, $CH_2\text{-}C\equiv CCH_2\text{-}Cl$ .

Besonders bevorzugte Verbindungen der Formel (I) sind solche, worin $R^1$ $(C_1\text{-}C_4)$-Alkyl, das wie oben beschrieben substituiert ist, oder $(C_3\text{-}C_4)$-Alkenyl, das wie oben beschrieben substituiert sein kann oder Propargyl, insbesondere Allyl oder Propargyl; $n = 0$; $R^3$ Wasserstoff, $R^4$ einen Rest der Formel

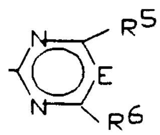

und $R^5$ und $R^6$ unabhängig voneinander Chlor, Brom, $(C_1\text{-}C_4)$-Alkyl, $(C_1\text{-}C_4)$-Alkoxy, $OCF_2H$, $OCH_2CF_3$ oder $CF_3$, insbesondere $(C_1\text{-}C_2)$Alkyl oder $(C_1\text{-}C_2)$Alkoxy, bedeuten, sowie deren Salze.

Weiterer Gegenstand der vorliegenden Erfindung sind Verfahren zur Herstellung von Verbindungen der allgemeinen Formel (I), dadurch gekennzeichnet, daß man

(a) eine Verbindung der Formel (II)

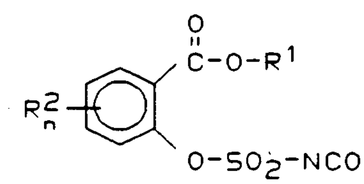

(II)

mit einer Verbindung der Formel (III)

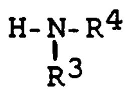

(III)

umsetzt, oder

(b) eine Verbindung der Formel (IV)

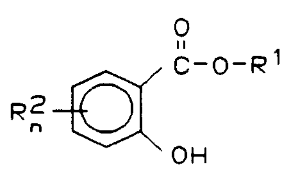

(IV)

mit einem Chlorsulfonylharnstoff der Formel (V)

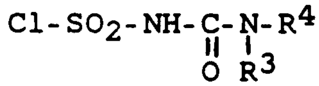 (V)

umsetzt, oder

(c) eine substituierte Benzoesäure der Formel (VI)

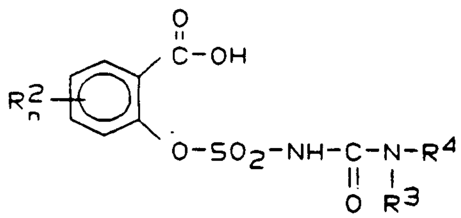 (VI)

mit einem Alkylierungsreagens der Formel (VII)

$R^1$-X (VII),

wobei X für eine nucleofuge Abgangsgruppe wie z. B. Halogen, Alkyl-$SO_2$-O- oder Tosyl steht, umsetzt.

Die Umsetzung der Verbindungen der Formeln (II) und (III) erfolgt vorzugsweise in inerten aprotischen Lösungsmitteln, wie z. B. Acetonitril, Dichlormethan, Toluol, Tetrahydrofuran oder Dioxan bei Temperaturen zwischen 0 ° C und der Siedetemperatur des Lösungsmittels.

Die Phenoxysulfonylisocyanate der Formel (II) lassen sich nach im Prinzip bekannten Verfahren aus den entsprechenden Salicylsäureestern der Formel (IV) und Chlorsulfonylisocyanat in einfacher Weise herstellen (vgl. G. Lohaus, Chem. Ber. 105, 2791 (1972)).

Die Ausgangsstoffe der Formel (III) sind bekannt oder lassen sich nach im Prinzip bekannten Verfahren herstellen, z. B. durch Cyclisierung entsprechender Guanidinderivate mit entsprechend substituierten 1,3-Diketonen, vergleiche z. B. "The Chemistry of Heterocyclic Compounds", Vol. XVI (1962) and Supplement I (1970), oder durch Derivatisierung von Cyanurchlorid, vgl. z. B. "The Chemistry of Heterocyclic Compounds", L. Rapaport: "s-Triazines and Derivates" (1959).

Die Umsetzung der Verbindungen (IV) mit den Chlorsulfonylharnstoffen (V) führt man vorzugsweise in inerten Lösungsmitteln wie z. B. Dichlormethan, Tetrahydrofuran, Dioxan oder Dimethoxyethan bei Temperaturen zwischen -10 ° C und 80 ° C in Gegenwart einer Base als HCl-bindendes Agens durch. Als Basen können Alkali- oder Erdalkalicarbonate bzw. -bicarbonate wie z. B. $K_2CO_3$, $NaHCO_3$, $Na_2CO_3$ oder tertiäre Amine wie z. B. Pyridin oder Triethylamin eingesetzt werden.

Die Salicylsäureester (IV) sind literaturbekannt oder können nach literaturbekannten Verfahren hergestellt werden. Die Chlorsulfonylharnstoffe (V) sind aus den Aminen der Formel (III) und Chlorsulfonylisocyanat zugänglich (EP-A 141 199).

Die Umsetzung der Benzoesäuren (VI) mit den Reagentien der Formel (VII) wird in inerten Lösungsmitteln wie z. B. Dimethylformamid oder Dimethylsulfoxid, vorzugsweise in Gegenwart einer Hilfsbase wie z. B. Triethylamin oder Tetramethylammoniumhydroxidpentahydrat, bei Temperaturen zwischen 0 ° C und der Siedetemperatur des Lösungsmittels durchgeführt.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöckchen oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im Einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z. B. Avena, Lolium, Alopecurus, Phalaris, Echinochloa, Digitaria, Setaria etc. sowie Cyperusarten aus der annuellen Gruppe und auf seiten der perennierenden Spezies Agropyron, Cynodon, Imperata sowie Sorghum etc. und auch ausdauernde Cyperusarten gut erfaßt.

Bei dikotylen Unkrautarten erstreckt sich das Wirkungsspektrum auf Arten wie z. B. Galium, Viola, Veronica, Lamium, Stellaria, Amaranthus, Sinapis, Ipomoea, Matricaria, Abutilon, Sida etc. auf der annuellen

Seite sowie Convolvulus, Cirsium, Rumex, Artemisia etc. bei den Perennierenden.

Unter den spezifischen Kulturbedingungen im Reis vorkommenden Unkräuter wie z. B. Sagittaria, Alisma, Eleocharis, Scirpus, Cyperus etc. werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis vier Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf diese Weise eine für die Kulturpflanzen schädliche Unkrautkonkurrenz sehr früh und nachhaltig durch den Einsatz der neuen erfindungsgemäßen Verbindungen beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrüber, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Verbindungen wachstumsregulatorische Eigenschaften bei Kulturpflanzen auf. Sie greifen regulierend in den pflanzeneigenen Stoffwechsel ein und können damit zur Ernteerleichterung wie z.B. durch Auslösen von Desikkation, Abszission und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Mittel können als Spritzpulver, emulgierbaren Konzentrate, versprühbare Lösungen, Stäubemittel, Beizmittel, Dispersionen, Granulate oder Mikrogranulate in den üblichen Zubereitungen angewendet werden.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel, z.B. ligninsulfonsaures Natrium, 2,2′-dinaphthylmethan-6,6′-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentrationen mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

Die Erfindung wird durch nachstehende Beispiele näher erläutert.

**Formulierungsbeispiele**

A. Ein Stäubemittel wird erhalten, indem man 10 Gewichtsteile Wirkstoff und 90 Gewichtsteile Talkum oder Inertstoff mischt und in einer Schlagmühle zerkleinert.

B. Ein in Wasser leicht dispergierbares, benetzbares Pulver wird erhalten, indem man 25 Gewichtsteile Wirkstoff, 64 Gewichtsteile kaolinhaltigen Quarz als Inerstoff, 10 Gewichtsteile ligninsulfonsaures Kalium und 1 Gewichtsteil oleoylmethyltaurinsaures Natrium als Netz-und Dispergiermittel mischt und in seiner Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z.B. ca. 255 bis über 377 °C) mischt und in einer Reibkugelmühle auf eine Feinheit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 AeO) als Emulgator.

**Chemische Beispiele**

Beispiel 1

2-Isocyanatosulfonyloxy-3-methyl-benzoesäureallylester

Zu einer Lösung von 4,8 g (0,025 mol) 3-Methylsalicylsäureallylester in 25 ml abs. Toluol tropft man bei 25 °C eine Lösung von 3,7 g (0,026 mol) Chlorsulfonylisocyanat in 25 ml abs. Toluol. Nach Beendigung des Zutropfens steigert man die Temperatur langsam auf 110 °C und erhitzt 6 h unter Rückfluß. Man kühlt ab und entfernt das Lösungsmittel am Rotationsverdampfer. Das zurückbleibende gelbe Öl (7,5 g ≙ 100 % d. Th.) wird ohne weitere Reinigung eingesetzt.

Beispiel 2

2-[3-(4-Methoxy-6-methylpyrimidin-2-yl)ureidosulfonyloxy]-3-methylbenzoesäureallylester

Zu 3,5 g (0,025 mol) 2-Amino-4-methoxy-6-methylpyrimidin in 15 ml Dichlormethan tropft man bei 0 °C eine Lösung aus 7,5 g (0,025 mol) des Produkts aus Beispiel 1 in 20 ml Dichlormethan. Man läßt auf Raumtemperatur kommen und rührt 24 h nach. Die Reaktionslösung wird mit 100 ml Dichlormethan verdünnt und mit 50 ml 1 N Salzsäure und 50 ml Wasser gewaschen. Man trocknet die organische Phase mit Natriumsulfat und entfernt das Lösungsmittel am Rotationsverdampfer. Das hinterbleibende viskose Öl wird durch Verreiben mit 1-Chlorbutan kristallisiert. Man erhält 7,4 g (68 % d. Th.)
2-[3-(4-Methoxy-6-methylpyrimidin-2-yl)ureidosulfonyloxy]-3-methylbenzoesäureallylester vom Schmp. 127 - 128 °C.

Beispiel 3

2-[3-(4,6-Dimethoxypyrimidin-2-yl)ureidosulfonyloxy]benzoesäure-2-chlorethylester

2,02 g (0,013 mol 2-Amino-4,6-dimethoxypyrimidin werden in 80 ml Dichlormethan gelöst und bei 0°C mit 4,28 g (0,014 mol) 2-Isocyanatosulfonyloxy-benzoesäure-2-chlorethylester - gelöst in 20 ml Dichlormethan - versetzt. Nach 18-stündigem Rühren bei Raumtemperatur wird das Reaktionsgemisch in 600 ml n-Heptan eingerührt. Man erhält auf diese Weise 4,72 g (79 % d. Th.)
2-[3-(4,6-Dimethoxypyrimidin-2-yl)ureidosulfonyloxy]benzoesäure-2-chlorethylester vom Schmp. 148 - 150°C.

Beispiel 4

2-[3-(4,6-Dimethoxypyrimidin-2-yl)ureidosulfonyloxy]benzoesäureallylester

1,55 g (0,01 mol) 2-Amino-4,6-dimethoxypyrimidin werden in 100 ml Dichlormethan gelöst und bei 0°C mit 3,40 g (0,012 mol) 2-Isocyanatosulfonyloxy-benzoesäureallylester - in 20 ml Dichlormethan gelöst - versetzt. Nach 18-stündigem Rühren bei Raumtemperatur erhält man durch Absaugen 2,54 g (58 % d. Th.)
2-[3-(4,6-Dimethoxypyrimidin-2-yl)-ureidosulfonyloxy]-benzoesäureallylester vom Schmp. 110 - 112°C.

Die nachfolgenden Verbindungen lassen sich auf analoge Weise herstellen.

**Tabellen 1a - c:**

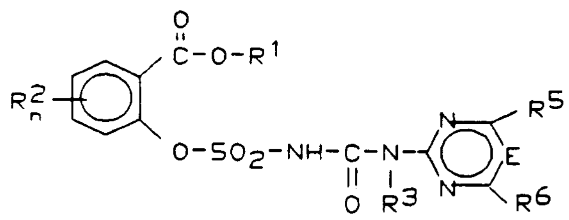

**Tabelle 1a: $(R^2)_n$ = H; $R^3$ = H**

| Bsp. Nr. | $R^1$ | $R^5$ | $R^6$ | E | Fp. [°C] |
|---|---|---|---|---|---|
| 5 | $CH_2CH_2Cl$ | $CH_3$ | $CH_3$ | CH | Öl |
| 6 | " | $OCH_3$ | $CH_3$ | CH | 56-60 |
| 7 | " | $CH_3$ | $CH_3$ | N | |
| 8 | " | $OCH_3$ | $CH_3$ | N | |
| 9 | " | $OCH_3$ | $OCH_3$ | N | |
| 10 | " | $OCH_3$ | Cl | CH | 132-135 |
| 11 | " | $OCF_2H$ | $CH_3$ | CH | |
| 12 | " | $OCF_2H$ | $OCF_2H$ | CH | |
| 13 | " | $OCH_3$ | Br | CH | |
| 14 | " | $CH_3$ | Cl | CH | |
| 15 | " | $OCH_3$ | H | CH | |
| 16 | " | $OCH_3$ | $NHCH_3$ | CH | |
| 17 | " | $OCH_3$ | $NHCH_3$ | N | |
| 18 | " | $CH_3$ | $NHCH_3$ | CH | |
| 19 | " | $CH_3$ | $NHCH_3$ | N | |
| 20 | " | $OCH_3$ | $SCH_3$ | CH | |
| 21 | " | $OCH_3$ | $OC_2H_5$ | CH | |
| 22 | " | $OCH_3$ | $OC_3H_7$ | CH | |
| 23 | " | $OCH_3$ | $OC_2H_5$ | N | |
| 24 | " | Cl | $OC_2H_5$ | CH | |
| 25 | " | $OC_2H_5$ | $OC_2H_5$ | CH | |
| 26 | " | $C_2H_5$ | $OCH_3$ | CH | |
| 27 | " | $CF_3$ | $OCH_3$ | CH | |

**Tabelle 1a: Fortsetzung**

| Bsp. Nr. | $R^1$ | $R^5$ | $R^6$ | E | Fp. |
|---|---|---|---|---|---|
| 28 | $CH_2CH_2Cl$ | $OCH_2CF_3$ | $CH_3$ | CH | |
| 29 | " | $OCH_2CF_3$ | $OCH_3$ | CH | |
| 30 | " | $OCH_2CF_3$ | $OCH_2CF_3$ | CH | |
| 31 | " | $OCH_2CF_3$ | $NHCH_3$ | CH | |
| 32 | " | $OCH_2CF_3$ | $OCH_3$ | N | |
| 33 | " | $OCH_2CF_3$ | $NHCH_3$ | N | |
| 34 | " | $OCH_3$ | $NHC_2H_5$ | CH | |
| 35 | " | $OCH_2CF_3$ | $NHC_2H_5$ | CH | |
| 36 | " | $OCH_3$ | $N(CH_3)_2$ | CH | |
| 37 | " | $OCH_3$ | $CH(OCH_3)_2$ | CH | |
| 38 | $CH_2CH_2OCH_3$ | $CH_3$ | $CH_3$ | CH | |
| 39 | " | $OCH_3$ | $CH_3$ | CH | |
| 40 | " | $CH_3$ | $CH_3$ | N | |
| 41 | " | $OCH_3$ | $CH_3$ | N | |
| 42 | " | $OCH_3$ | $OCH_3$ | N | |
| 43 | " | $OCH_3$ | Cl | CH | |
| 44 | " | $OCF_2H$ | $CH_3$ | CH | |
| 45 | " | $OCF_2H$ | $OCF_2H$ | CH | |
| 46 | " | $OCH_3$ | Br | CH | |
| 47 | " | $CH_3$ | Cl | CH | |
| 48 | " | $OCH_3$ | H | CH | |
| 49 | " | $OCH_3$ | $NHCH_3$ | CH | |
| 50 | " | $OCH_3$ | $NHCH_3$ | N | |
| 51 | " | $CH_3$ | $NHCH_3$ | CH | |
| 52 | " | $CH_3$ | $NHCH_3$ | N | |
| 53 | " | $OCH_3$ | $SCH_3$ | CH | |
| 54 | " | $OCH_3$ | $OC_2H_5$ | CH | |
| 55 | " | $OCH_3$ | $OC_3H_7$ | CH | |
| 56 | " | $OCH_3$ | $OC_2H_5$ | N | |
| 57 | " | Cl | $OC_2H_5$ | CH | |
| 58 | " | $OC_2H_5$ | $OC_2H_5$ | CH | |

**Tabelle 1a: Fortsetzung**

| Bsp. Nr. | $R^1$ | $R^5$ | $R^6$ | E | Fp. |
|---|---|---|---|---|---|
| 59 | $CH_2CH_2OCH_3$ | $C_2H_5$ | $OCH_3$ | CH | |
| 60 | " | $CF_3$ | $OCH_3$ | CH | |
| 61 | " | $OCH_2CF_3$ | $CH_3$ | CH | |
| 62 | " | $OCH_2CF_3$ | $OCH_3$ | CH | |
| 63 | " | $OCH_2CF_3$ | $OCH_2CF_3$ | CH | |
| 64 | " | $OCH_2CF_3$ | $NHCH_3$ | CH | |
| 65 | " | $OCH_2CF_3$ | $OCH_3$ | N | |
| 66 | " | $OCH_2CF_3$ | $NHCH_3$ | N | |
| 67 | " | $OCH_3$ | $OCH_3$ | CH | 131-133 |
| 68 | " | $OCH_2CF_3$ | $NHC_2H_5$ | CH | |
| 69 | " | $OCH_3$ | $N(CH_3)_2$ | CH | |
| 70 | " | $OCH_3$ | $CH(OCH_3)_2$ | CH | |
| 71 | $CH_2CH=CH_2$ | $CH_3$ | $CH_3$ | CH | |
| 72 | " | $OCH_3$ | $CH_3$ | CH | |
| 73 | " | $CH_3$ | $CH_3$ | N | |
| 74 | " | $OCH_3$ | $CH_3$ | N | Öl |
| 75 | " | $OCH_3$ | $OCH_3$ | N | |
| 76 | " | $OCH_3$ | Cl | CH | |
| 77 | " | $OCF_2H$ | $CH_3$ | CH | |
| 78 | " | $OCF_2H$ | $OCF_2H$ | CH | |
| 79 | " | $OCH_3$ | Br | CH | |
| 80 | " | $CH_3$ | Cl | CH | |
| 81 | " | $OCH_3$ | H | CH | |
| 82 | " | $OCH_3$ | $NHCH_3$ | CH | |
| 83 | " | $OCH_3$ | $NHCH_3$ | N | |
| 84 | " | $CH_3$ | $NHCH_3$ | CH | |
| 85 | " | $CH_3$ | $NHCH_3$ | N | |
| 86 | " | $OCH_3$ | $SCH_3$ | CH | |
| 87 | " | $OCH_3$ | $OC_2H_5$ | CH | |
| 88 | " | $OCH_3$ | $OC_3H_7$ | CH | |
| 89 | " | $OCH_3$ | $OC_2H_5$ | N | |

**Tabelle 1a: Fortsetzung**

| Bsp. Nr. | $R^1$ | $R^5$ | $R^6$ | E | Fp. |
|---|---|---|---|---|---|
| 90 | $CH_2CH=CH_2$ | Cl | $OC_2H_5$ | CH | |
| 91 | " | $OC_2H_5$ | $OC_2H_5$ | CH | Harz |
| 92 | " | $C_2H_5$ | $OCH_3$ | CH | |
| 93 | " | $CF_3$ | $OCH_3$ | CH | |
| 94 | " | $OCH_2CF_3$ | $CH_3$ | CH | |
| 95 | " | $OCH_2CF_3$ | $OCH_3$ | CH | |
| 96 | " | $OCH_2CF_3$ | $OCH_2CF_3$ | CH | |
| 97 | " | $OCH_2CF_3$ | $NHCH_3$ | CH | |
| 98 | " | $OCH_2CF_3$ | $OCH_3$ | N | |
| 99 | " | $OCH_2CF_3$ | $NHCH_3$ | N | |
| 100 | " | $OCH_3$ | $NHC_2H_5$ | CH | |
| 101 | " | $OCH_2CF_3$ | $NHC_2H_5$ | CH | |
| 102 | " | $OCH_3$ | $N(CH_3)_2$ | CH | |
| 103 | " | $OCH_3$ | $CH(OCH_3)_2$ | CH | |
| 104 | $CH_2CF_3$ | $OCH_3$ | $OCH_3$ | CH | 139-140 |
| 105 | " | $CH_3$ | $CH_3$ | CH | |
| 106 | " | $OCH_3$ | $CH_3$ | CH | |
| 107 | " | $CH_3$ | $CH_3$ | N | |
| 108 | " | $OCH_3$ | $CH_3$ | N | |
| 109 | " | $OCH_3$ | $OCH_3$ | N | |
| 110 | " | $OCH_3$ | Cl | CH | |
| 111 | " | $OCF_2H$ | $CH_3$ | CH | |
| 112 | " | $OCF_2H$ | $OCF_2H$ | CH | |
| 113 | " | $OCH_3$ | Br | CH | |
| 114 | " | $CH_3$ | Cl | CH | |
| 115 | " | $OCH_3$ | H | CH | |
| 116 | " | $OCH_3$ | $NHCH_3$ | CH | |
| 117 | " | $OCH_3$ | $NHCH_3$ | N | |
| 118 | " | $CH_3$ | $NHCH_3$ | CH | |
| 119 | " | $CH_3$ | $NHCH_3$ | N | |
| 120 | " | $OCH_3$ | $SCH_3$ | CH | |

**Tabelle 1a: Fortsetzung**

| Bsp. Nr. | $R^1$ | $R^5$ | $R^6$ | E | Fp. |
|---|---|---|---|---|---|
| 121 | $CH_2CF_3$ | $OCH_3$ | $OC_2H_5$ | CH | |
| 122 | " | $OCH_3$ | $OC_3H_7$ | CH | |
| 123 | " | $OCH_3$ | $OC_2H_5$ | N | |
| 124 | " | Cl | $OC_2H_5$ | CH | |
| 125 | " | $OC_2H_5$ | $OC_2H_5$ | CH | |
| 126 | " | $C_2H_5$ | $OCH_3$ | CH | |
| 127 | " | $CF_3$ | $OCH_3$ | CH | |
| 128 | " | $OCH_2CF_3$ | $CH_3$ | CH | |
| 129 | " | $OCH_2CF_3$ | $OCH_3$ | CH | |
| 130 | " | $OCH_2CF_3$ | $OCH_2CF_3$ | CH | |
| 131 | " | $OCH_2CF_3$ | $NHCH_3$ | CH | |
| 132 | " | $OCH_2CF_3$ | $OCH_3$ | N | |
| 133 | " | $OCH_2CF_3$ | $NHCH_3$ | N | |
| 134 | " | $OCH_3$ | $NHC_2H_5$ | CH | |
| 135 | " | $OCH_2CF_3$ | $NHC_2H_5$ | CH | |
| 136 | " | $OCH_3$ | $N(CH_3)_2$ | CH | |
| 137 | " | $OCH_3$ | $CH(OCH_3)_2$ | CH | |
| 138 | $CH_2C{\equiv}CH$ | $OCH_3$ | $OCH_3$ | CH | 124-125 |
| 139 | " | $OCH_3$ | $CH_3$ | CH | 56- 58 |
| 140 | " | $OCH_3$ | $CH_3$ | N | 60- 62 |
| 141 | " | $OCH_3$ | $OCH_3$ | N | 144 |
| 142 | " | $OCH_3$ | Cl | CH | |
| 143 | " | $OCF_2H$ | $CH_3$ | CH | |
| 144 | " | $OCF_2H$ | $OCF_2H$ | CH | |
| 145 | " | $OCH_3$ | $NHCH_3$ | CH | |
| 146 | " | Cl | $OC_2H_5$ | CH | |
| 147 | " | $OCH_2CF_3$ | $OCH_3$ | CH | |
| 148 | " | $OCH_2CF_3$ | $OCH_3$ | N | |
| 149 | $CH_2CH{=}CHCl$ | $OCH_3$ | $OCH_3$ | CH | |
| 150 | " | $OCH_3$ | $CH_3$ | CH | |
| 151 | " | $OCH_3$ | $CH_3$ | N | |

**Tabelle 1a: Fortsetzung**

| Bsp. Nr. | $R^1$ | $R^5$ | $R^6$ | E | Fp. |
|---|---|---|---|---|---|
| 152 | $CH_2CH=CHCl$ | $OCH_3$ | $OCH_3$ | N | |
| 153 | " | $OCH_3$ | Cl | CH | |
| 154 | " | $OCF_2H$ | $CH_3$ | CH | |
| 155 | " | $OCF_2H$ | $OCF_2H$ | CH | |
| 156 | " | $OCH_3$ | $NHCH_3$ | CH | |
| 157 | " | Cl | $OC_2H_5$ | CH | |
| 158 | " | $OCH_2CF_3$ | $OCH_3$ | CH | |
| 159 | " | $OCH_2CF_3$ | $OCH_3$ | N | |
| 160 | $CH_2CCl=CCl_2$ | $OCH_3$ | $OCH_3$ | CH | |
| 161 | " | $OCH_3$ | $CH_3$ | CH | |
| 162 | " | $OCH_3$ | $CH_3$ | N | |
| 163 | " | $OCH_3$ | $OCH_3$ | N | |
| 164 | " | $OCH_3$ | Cl | CH | |
| 165 | " | $OCF_2H$ | $CH_3$ | CH | |
| 166 | " | $OCF_2H$ | $OCF_2H$ | CH | |
| 167 | " | $OCH_3$ | $NHCH_3$ | CH | |
| 168 | " | Cl | $OC_2H_5$ | CH | |
| 169 | " | $OCH_2CF_3$ | $OCH_3$ | CH | |
| 170 | " | $OCH_2CF_3$ | $OCH_3$ | N | |
| 171 | $CH_2CH=CHCH_3$ | $OCH_3$ | $OCH_3$ | CH | |
| 172 | " | $OCH_3$ | $CH_3$ | CH | |
| 173 | " | $OCH_3$ | $CH_3$ | N | |
| 174 | " | $OCH_3$ | $OCH_3$ | N | |
| 175 | " | $OCH_3$ | Cl | CH | |
| 176 | " | $OCF_2H$ | $CH_3$ | CH | |
| 177 | " | $OCF_2H$ | $OCF_2H$ | CH | |
| 178 | " | $OCH_3$ | $NHCH_3$ | CH | |
| 179 | " | Cl | $OC_2H_5$ | CH | |
| 180 | " | $OCH_2CF_3$ | $OCH_3$ | CH | |
| 181 | " | $OCH_2CF_3$ | $OCH_3$ | N | |
| 182 | $CH_2C{\equiv}CCH_3$ | $OCH_3$ | $OCH_3$ | CH | |

**Tabelle 1a: Fortsetzung**

| Bsp. Nr. | $R^1$ | $R^5$ | $R^6$ | E | Fp. |
|---|---|---|---|---|---|
| 183 | $CH_2C{\equiv}CCH_3$ | $OCH_3$ | $CH_3$ | CH | |
| 184 | " | $OCH_3$ | $CH_3$ | N | |
| 185 | " | $OCH_3$ | $OCH_3$ | N | |
| 186 | " | $OCH_3$ | Cl | CH | |
| 187 | " | $OCF_2H$ | $CH_3$ | CH | |
| 188 | " | $OCF_2H$ | $OCF_2H$ | CH | |
| 189 | " | $OCH_3$ | $NHCH_3$ | CH | |
| 190 | " | Cl | $OC_2H_5$ | CH | |
| 191 | " | $OCH_2CF_3$ | $OCH_3$ | CH | |
| 192 | " | $OCH_2CF_3$ | $OCH_3$ | N | |
| 193 | $CH_2CH_2Br$ | $OCH_3$ | $OCH_3$ | CH | |
| 194 | " | $OCH_3$ | $CH_3$ | CH | |
| 195 | " | $OCH_3$ | $CH_3$ | N | |
| 196 | " | $OCH_3$ | $OCH_3$ | N | |
| 197 | " | $OCH_3$ | Cl | CH | |
| 198 | " | $OCF_2H$ | $CH_3$ | CH | |
| 199 | " | $OCF_2H$ | $OCF_2H$ | CH | |
| 200 | " | $OCH_3$ | $NHCH_3$ | CH | |
| 201 | " | Cl | $OC_2H_5$ | CH | |
| 202 | " | $OCH_2CF_3$ | $OCH_3$ | CH | |
| 203 | " | $OCH_2CF_3$ | $OCH_3$ | N | |
| 204 | $CH_2CH(OCH_3)_2$ | $OCH_3$ | $OCH_3$ | CH | |
| 205 | " | $OCH_3$ | $CH_3$ | CH | |
| 206 | " | $OCH_3$ | $CH_3$ | N | |

**Tabelle 1b: $R^3$ = H**

| Bsp. Nr. | $R^1$ | $(R^2)_n$ | $R^5$ | $R^6$ | E | Fp. |
|---|---|---|---|---|---|---|
| 207 | $CH_2CH_2Cl$ | 3-$CH_3$ | $OCH_3$ | $OCH_3$ | CH | 154-156 |
| 208 | " | 3-$CH_3$ | $OCH_3$ | $CH_3$ | N | |
| 209 | " | 6-$CH_3$ | $OCH_3$ | $OCH_3$ | CH | |
| 210 | " | 6-$CH_3$ | $OCH_3$ | Cl | CH | |
| 211 | $CH_2CH_2OCH_3$ | 3-$CH_3$ | $OCH_3$ | $OCH_3$ | CH | |
| 212 | " | 3-$CH_3$ | $OCH_3$ | $CH_3$ | CH | |
| 213 | $CH_2CH{=}CH_2$ | 3-$CH_3$ | $OCH_3$ | $OCH_2CF_3$ | CH | |
| 214 | " | 3-$CH_3$ | $OCH_3$ | $CH_3$ | N | |
| 215 | " | 3-$CH_3$ | $OCH_3$ | $OCH_3$ | CH | 126-127 |
| 216 | " | 3-$CH_3$ | $OCH_3$ | Cl | CH | |
| 217 | " | 3-$CH_3$ | $CH_3$ | $CH_3$ | CH | 107-110 |
| 218 | " | 3-$CH_3$ | $OCH_3$ | Cl | N | |
| 219 | " | 3-$CH_3$ | $OCH_3$ | $OCH_2CF_3$ | N | |
| 220 | " | 3-$CH_3$ | $OC_2H_5$ | $NHCH_3$ | N | |
| 221 | $CH_2CF_3$ | 3-$CH_3$ | $OCH_3$ | $OCH_3$ | CH | |
| 222 | " | 3-$CH_3$ | $OCH_3$ | Cl | CH | |
| 223 | $CH_2C{\equiv}CH$ | 3-$CH_3$ | $OCH_3$ | $OCH_3$ | CH | 126 |
| 224 | " | 6-$CH_3$ | $OCF_2H$ | $OCF_2H$ | CH | |
| 225 | $CH_2CH{=}CHCl$ | 3-$CH_3$ | $OCH_3$ | $OCH_3$ | CH | |
| 226 | " | 6-$CH_3$ | $OCH_3$ | $CH_3$ | N | |
| 227 | $CH_2CH_2Cl$ | 3-$OCH_3$ | $OCH_3$ | $OCH_3$ | CH | |
| 228 | " | 3-$OCH_3$ | $OCH_3$ | Cl | CH | |
| 229 | " | 6-$OCH_3$ | $OCH_3$ | $OCH_3$ | CH | |
| 230 | " | 6-$OCH_3$ | $OCH_2CF_3$ | $OCH_3$ | CH | |
| 231 | $CH_2CH_2OCH_3$ | 3-$OCH_3$ | $OCH_3$ | $OCH_3$ | CH | |
| 232 | " | 3-$OCH_3$ | $OCH_3$ | $CH_3$ | N | |
| 233 | $CH_2CH{=}CH_2$ | 3-$OCH_3$ | $CH_3$ | $CH_3$ | CH | 151-152 |
| 234 | " | 3-$OCH_3$ | $OCH_3$ | $CH_3$ | CH | 132-133 |
| 235 | " | 3-$OCH_3$ | $OCH_3$ | $OCH_3$ | CH | 138-139 |
| 236 | " | 3-$OCH_3$ | $OCH_2CF_3$ | $OCH_3$ | CH | |
| 237 | " | 3-$OCH_3$ | $OCH_3$ | Cl | CH | |
| 238 | " | 3-$OCH_3$ | $OCH_3$ | $CH_3$ | N | 135-136 |

**Tabelle 1b: Fortsetzung**

| Bsp. Nr. | $R^1$ | $(R^2)_n$ | $R^5$ | $R^6$ | E | Fp. |
|---|---|---|---|---|---|---|
| 239 | $CH_2CH=CH_2$ | $3-OCH_3$ | $OCH_3$ | Cl | N | |
| 240 | " | $3-OCH_3$ | $OCH_3$ | $OCH_2CF_3$ | N | |
| 241 | " | $3-OCH_3$ | $OC_2H_5$ | $NHCH_3$ | N | |
| 242 | $CH_2CF_3$ | $3-OCH_3$ | $OCH_3$ | Cl | CH | |
| 243 | " | $3-OCH_3$ | $OCH_3$ | $OCH_3$ | CH | |
| 244 | " | $6-OCH_3$ | $OCH_3$ | $CH_3$ | CH | |
| 245 | $CH_2CH_2Br$ | $3-OCH_3$ | $OCH_3$ | $OCH_3$ | CH | |
| 246 | " | $6-OCH_3$ | $OCH_3$ | $CH_3$ | N | |
| 247 | $CH_2CH_2Cl$ | 3-Cl | $OCH_3$ | $OCH_3$ | CH | |
| 248 | " | 3-Cl | $OCH_3$ | Cl | CH | |
| 249 | " | 4-Cl | $OCH_3$ | $OCH_3$ | CH | |
| 250 | " | 4-Cl | $OCF_2H$ | $CH_3$ | CH | |
| 251 | " | 5-Cl | $OCH_3$ | $OCH_3$ | CH | |
| 252 | " | 5-Cl | $OCH_3$ | $CH_3$ | N | |
| 253 | " | 6-Cl | $OCH_3$ | $OCH_3$ | CH | |
| 254 | " | 6-Cl | $OCH_3$ | Cl | CH | |
| 255 | $CH_2CH_2OCH_3$ | 3-Cl | $OCH_3$ | $OCH_3$ | CH | |
| 256 | " | 3-Cl | $OCF_2H$ | $OCF_2H$ | CH | |
| 257 | " | 4-Cl | $OCH_3$ | $OCH_3$ | CH | |
| 258 | " | 4-Cl | $OCH_3$ | $CH_3$ | N | |
| 259 | " | 5-Cl | $OCH_3$ | $OCH_3$ | CH | |
| 260 | " | 5-Cl | $OCH_3$ | Cl | CH | |
| 261 | " | 6-Cl | $OCH_3$ | $OCH_3$ | CH | |
| 262 | $CH_2CH=CH_2$ | 4-Cl | $OCH_2CF_3$ | $OCH_3$ | CH | |
| 263 | " | 4-Cl | $OCH_3$ | $OCH_3$ | CH | 104-106 |
| 264 | " | 4-Cl | $OCH_3$ | $CH_3$ | N | |
| 265 | " | 4-Cl | $CH_3$ | $CH_3$ | CH | 93- 95 |
| 266 | " | 4-Cl | $OCH_3$ | Cl | CH | |
| 267 | " | 4-Cl | $OCH_3$ | $CH_3$ | CH | 121-124 |
| 268 | " | 4-Cl | $OCH_2CF_3$ | $OCH_3$ | N | |
| 269 | " | 4-Cl | $OCH_3$ | Cl | N | |
| 270 | " | 4-Cl | $OC_2H_5$ | $NHCH_3$ | N | |

Tabelle 1b: Fortsetzung

| Bsp. Nr. | $R^1$ | $(R^2)_n$ | $R^5$ | $R^6$ | E | Fp. |
|---|---|---|---|---|---|---|
| 271 | $CH_2CH=CH_2$ | 5-Cl | $OCH_3$ | $OCH_3$ | CH | 138 |
| 272 | " | 5-Cl | $OCH_3$ | $CH_3$ | N | 136 |
| 273 | " | 5-Cl | $CH_3$ | $CH_3$ | CH | 110-112 |
| 274 | " | 5-Cl | $CH_3$ | Cl | CH | 108-109 |
| 275 | " | 5-Cl | $OCH_3$ | $CH_3$ | CH | |
| 276 | " | 5-Cl | $OCH_3$ | $OCH_2CF_3$ | CH | |
| 277 | " | 5-Cl | $OCH_3$ | Cl | N | |
| 278 | " | 5-Cl | $OCH_3$ | $OCH_2CF_3$ | N | |
| 279 | $CH_2CF_3$ | 3-Cl | $OCH_3$ | $OCH_3$ | CH | |
| 280 | " | 4-Cl | $OCH_3$ | Cl | CH | |
| 281 | " | 5-Cl | $OCH_3$ | $OCH_3$ | CH | |
| 282 | " | 6-Cl | $OCF_2H$ | $CH_3$ | CH | |
| 283 | $CH_2CH=CHCH_3$ | 3-Cl | $OCH_3$ | $OCH_3$ | CH | |
| 284 | " | 4-Cl | $OCH_3$ | $CH_3$ | N | |
| 285 | " | 5-Cl | $OCH_3$ | $OCH_3$ | CH | |
| 286 | " | 6-Cl | $OCH_3$ | Cl | CH | |
| 287 | $CH_2CH_2Cl$ | 3-F | $OCH_3$ | $OCH_3$ | CH | |
| 288 | " | 3-F | $OCF_2H$ | $OCF_2H$ | CH | |
| 289 | " | 4-F | $OCH_3$ | $OCH_3$ | CH | |
| 290 | " | 4-F | $OCH_3$ | $CH_3$ | N | |
| 291 | " | 5-F | $OCH_3$ | $OCH_3$ | CH | |
| 292 | " | 5-F | $OCH_3$ | Cl | CH | |
| 293 | " | 6-F | $OCH_3$ | $OCH_3$ | CH | |
| 294 | " | 6-F | $OCH_2CF_3$ | $OCH_3$ | CH | |
| 295 | $CH_2CH=CH_2$ | 3-F | $OCH_3$ | $OCH_3$ | CH | 154 |
| 296 | " | 3-Cl | $OCH_3$ | $CH_3$ | N | |
| 297 | " | 4-F | $OCH_3$ | $OCH_3$ | CH | 93 |
| 298 | " | 4-F | $OCH_3$ | Cl | CH | |
| 299 | " | 5-F | $OCH_3$ | $OCH_3$ | CH | 145-146 |
| 300 | " | 5-F | $OCH_2CF_3$ | $OCH_3$ | N | 103-105 |
| 301 | " | 6-F | $OCH_3$ | $OCH_3$ | CH | |
| 302 | " | 6-Cl | $OCH_3$ | $OCH_3$ | N | |

**Tabelle 1b: Fortsetzung**

| Bsp. Nr. | $R^1$ | $(R^2)_n$ | $R^5$ | $R^6$ | E | Fp. |
|---|---|---|---|---|---|---|
| 303 | $CH_2CH_2OCH_3$ | 3-F | $OCH_3$ | $OCH_3$ | CH | |
| 304 | " | 3-F | $OCH_3$ | $CH_3$ | N | |
| 305 | " | 4-F | $OCH_3$ | $OCH_3$ | CH | |
| 306 | " | 4-F | $OCH_3$ | Cl | CH | |
| 307 | " | 5-F | $OCH_3$ | $OCH_3$ | CH | |
| 308 | " | 5-F | $OCH_3$ | $CH_3$ | CH | |
| 309 | " | 6-F | $OCH_3$ | $OCH_3$ | CH | |
| 310 | " | 6-F | $OCH_3$ | $CH_3$ | N | |
| 311 | $CH_2CF_3$ | 3-F | $OCH_3$ | $OCH_3$ | CH | |
| 312 | " | 3-F | $OCH_3$ | Cl | CH | |
| 313 | " | 4-F | $OCH_3$ | $OCH_3$ | CH | |
| 314 | " | 4-F | $OCF_2H$ | $CH_3$ | CH | |
| 315 | " | 5-F | $OCH_3$ | $OCH_3$ | CH | |
| 316 | " | 5-F | $OCH_3$ | $CH_3$ | N | |
| 317 | " | 6-F | $OCH_3$ | $OCH_3$ | CH | |
| 318 | " | 6-F | $OCH_3$ | Cl | CH | |
| 319 | $CH_2C\equiv CH$ | 3-F | $OCH_3$ | $OCH_3$ | CH | |
| 320 | " | 4-F | $OCF_2H$ | $OCF_2H$ | CH | |
| 321 | " | 5-F | $OCH_3$ | $OCH_3$ | CH | |
| 322 | " | 6-F | $OCH_3$ | $CH_3$ | N | |
| 323 | $CH_2CH{=}CHCl$ | 3-F | $OCH_3$ | $OCH_3$ | CH | |
| 324 | " | 4-F | $OCH_3$ | Cl | CH | |
| 325 | " | 5-F | $OCH_3$ | $OCH_3$ | CH | |
| 326 | " | 6-F | $OCH_2CF_3$ | $OCH_3$ | CH | |
| 327 | $CH_2CH_2Cl$ | $3-CF_3$ | $OCH_3$ | $OCH_3$ | CH | |
| 328 | " | $3-CF_3$ | $OCH_3$ | $CH_3$ | N | |
| 329 | " | $4-CF_3$ | $OCH_3$ | $OCH_3$ | CH | |
| 330 | " | $4-CF_3$ | $OCH_3$ | Cl | CH | |
| 331 | " | $5-CF_3$ | $OCH_3$ | $OCH_3$ | CH | |
| 332 | " | $5-CF_3$ | $OCH_2CF_3$ | $OCH_3$ | N | |
| 333 | " | $6-CF_3$ | $OCH_3$ | $OCH_3$ | CH | |
| 334 | " | $6-CF_3$ | $OCH_3$ | $OCH_3$ | N | |

**Tabelle 1b: Fortsetzung**

| Bsp. Nr. | $R^1$ | $(R^2)_n$ | $R^5$ | $R^6$ | E | Fp. |
|---|---|---|---|---|---|---|
| 335 | $CH_2CH_2OCH_3$ | 3-$CF_3$ | $OCH_3$ | $OCH_3$ | CH | |
| 336 | " | 3-$CF_3$ | $OCH_3$ | $CH_3$ | N | |
| 337 | " | 4-$CF_3$ | $OCH_3$ | $OCH_3$ | CH | |
| 338 | " | 4-$CF_3$ | $OCH_3$ | Cl | CH | |
| 339 | " | 5-$CF_3$ | $OCH_3$ | $OCH_3$ | CH | |
| 340 | " | 5-$CF_3$ | $OCH_3$ | $CH_3$ | CH | |
| 341 | " | 6-$CF_3$ | $OCH_3$ | $OCH_3$ | CH | |
| 342 | " | 6-$CF_3$ | $OCH_3$ | $CH_3$ | N | |
| 343 | $CH_2CF_3$ | 3-$CF_3$ | $OCH_3$ | $OCH_3$ | CH | |
| 344 | " | 3-$CF_3$ | $OCH_3$ | Cl | CH | |
| 345 | " | 4-$CF_3$ | $OCH_3$ | $OCH_3$ | CH | |
| 346 | " | 4-$CF_3$ | $OCF_2H$ | $CH_3$ | CH | |
| 347 | " | 5-$CF_3$ | $OCH_3$ | $OCH_3$ | CH | |
| 348 | " | 5-$CF_3$ | $OCH_3$ | $CH_3$ | N | |
| 349 | " | 6-$CF_3$ | $OCH_3$ | $OCH_3$ | CH | |
| 350 | " | 6-$CF_3$ | $OCH_3$ | Cl | CH | |
| 351 | $CH_2CH=CH_2$ | 3-$CF_3$ | $OCH_3$ | $OCH_3$ | CH | |
| 352 | " | 3-$CF_3$ | $OCF_2H$ | $OCF_2H$ | CH | |
| 353 | " | 4-$CF_3$ | $OCH_3$ | $OCH_3$ | CH | |
| 354 | " | 4-$CF_3$ | $OCH_3$ | $CH_3$ | N | |
| 355 | " | 5-$CF_3$ | $OCH_3$ | $OCH_3$ | CH | |
| 356 | " | 5-$CF_3$ | $OCH_3$ | Cl | CH | |
| 357 | " | 6-$CF_3$ | $OCH_3$ | $OCH_3$ | CH | |
| 358 | " | 6-$CF_3$ | $OCH_2CF_3$ | $OCH_3$ | CH | |
| 359 | $CH_2CH_2Br$ | 3-$CF_3$ | $OCH_3$ | $OCH_3$ | CH | |
| 360 | " | 4-$CF_3$ | $OCH_3$ | $CH_3$ | N | |
| 361 | " | 5-$CF_3$ | $OCH_3$ | $OCH_3$ | CH | |
| 362 | " | 6-$CF_3$ | $OCH_3$ | Cl | CH | |
| 363 | $CH_2C{\equiv}CCH_3$ | 3-$CF_3$ | $OCH_3$ | $OCH_3$ | CH | |
| 364 | " | 4-$CF_3$ | $OCH_2CF_3$ | $OCH_3$ | N | |
| 365 | " | 5-$CF_3$ | $OCH_3$ | $OCH_3$ | CH | |
| 366 | " | 6-$CF_3$ | $OCH_3$ | $OCH_3$ | N | |

**Tabelle 1b: Fortsetzung**

| Bsp. Nr. | $R^1$ | $(R^2)_n$ | $R^5$ | $R^6$ | E | Fp. |
|---|---|---|---|---|---|---|
| 367 | $CH_2CH_2OCH_3$ | 4-$OCF_2H$ | $OCH_3$ | $OCH_3$ | CH | |
| 368 | " | 4-$OCF_2H$ | $OCH_3$ | $CH_3$ | N | |
| 369 | $CH_2CH_2Cl$ | 4-$OCF_2H$ | $OCH_3$ | $OCH_3$ | CH | |
| 370 | " | 4-$OCF_2H$ | $OCH_3$ | Cl | CH | |
| 371 | $CH_2CH{=}CH_2$ | 4-$OCF_2H$ | $OCH_3$ | $OCH_3$ | CH | |
| 372 | " | 4-$OCF_2H$ | $OCH_3$ | $CH_3$ | CH | |
| 373 | $CH_2$-$CF_3$ | 4-$OCF_2H$ | $OCH_3$ | $OCH_3$ | CH | |
| 374 | " | 4-$OCF_2H$ | $OCH_3$ | $CH_3$ | N | |
| 375 | $CH_2CH_2Cl$ | 3-$NO_2$ | $OCH_3$ | $OCH_3$ | CH | |
| 376 | " | 3-$NO_2$ | $OCH_3$ | Cl | CH | |
| 377 | " | 4-$NO_2$ | $OCH_3$ | $OCH_3$ | CH | |
| 378 | " | 4-$NO_2$ | $OCF_2H$ | $CH_3$ | CH | |
| 379 | " | 5-$NO_2$ | $OCH_3$ | $OCH_3$ | CH | |
| 380 | " | 5-$NO_2$ | $OCH_3$ | $CH_3$ | N | |
| 381 | " | 6-$NO_2$ | $OCH_3$ | $OCH_3$ | CH | |
| 382 | " | 6-$NO_2$ | $OCH_3$ | Cl | CH | |
| 383 | $CH_2CH{=}CH_2$ | 3-$NO_2$ | $OCH_3$ | $OCH_3$ | CH | |
| 384 | " | 3-$NO_2$ | $OCF_2H$ | $OCF_2H$ | CH | |
| 385 | " | 4-$NO_2$ | $OCH_3$ | $OCH_3$ | CH | |
| 386 | " | 4-$NO_2$ | $OCH_3$ | $CH_3$ | N | |
| 387 | " | 5-$NO_2$ | $OCH_3$ | $OCH_3$ | CH | |
| 388 | " | 5-$NO_2$ | $OCH_3$ | Cl | CH | |
| 389 | " | 6-$NO_2$ | $OCH_3$ | $OCH_3$ | CH | |
| 390 | " | 6-$NO_2$ | $OCH_2CF_3$ | $OCH_3$ | CH | |
| 391 | $CH_2CH_2OCH_3$ | 3-$NO_2$ | $OCH_3$ | $OCH_3$ | CH | |
| 392 | " | 3-$NO_2$ | $OCH_3$ | $CH_3$ | N | |
| 393 | " | 4-$NO_2$ | $OCH_3$ | $OCH_3$ | CH | |
| 394 | " | 4-$NO_2$ | $OCH_3$ | Cl | CH | |
| 395 | " | 5-$NO_2$ | $OCH_3$ | $OCH_3$ | CH | |
| 396 | " | 5-$NO_2$ | $OCH_2CF_3$ | $OCH_3$ | N | |
| 397 | " | 6-$NO_2$ | $OCH_3$ | $OCH_3$ | CH | |
| 398 | " | 6-$NO_2$ | $OCH_3$ | $OCH_3$ | N | |

**Tabelle 1b: Fortsetzung**

| Bsp. Nr. | $R^1$ | $(R^2)_n$ | $R^5$ | $R^6$ | E | Fp. |
|---|---|---|---|---|---|---|
| 399 | $CH_2CF_3$ | 3-$NO_2$ | $OCH_3$ | $OCH_3$ | CH | |
| 400 | " | 3-$NO_2$ | $OCH_3$ | $CH_3$ | N | |
| 401 | " | 4-$NO_2$ | $OCH_3$ | $OCH_3$ | CH | |
| 402 | " | 4-$NO_2$ | $OCH_3$ | Cl | CH | |
| 403 | " | 5-$NO_2$ | $OCH_3$ | $OCH_3$ | CH | |
| 404 | " | 5-$NO_2$ | $OCH_3$ | $CH_3$ | CH | |
| 405 | " | 6-$NO_2$ | $OCH_3$ | $OCH_3$ | CH | |
| 406 | " | 6-$NO_2$ | $OCH_3$ | $CH_3$ | N | |
| 407 | $CH_2C{\equiv}CH$ | 3-$NO_2$ | $OCH_3$ | $OCH_3$ | CH | |
| 408 | " | 4-$NO_2$ | $OCH_3$ | Cl | CH | |
| 409 | " | 5-$NO_2$ | $OCH_3$ | $OCH_3$ | CH | |
| 410 | " | 6-$NO_2$ | $OCF_2H$ | $CH_3$ | CH | |
| 411 | $CH_2CH{=}CHCl$ | 3-$NO_2$ | $OCH_3$ | $OCH_3$ | CH | |
| 412 | " | 4-$NO_2$ | $OCH_3$ | $CH_3$ | N | |
| 413 | " | 5-$NO_2$ | $OCH_3$ | $OCH_3$ | CH | |
| 414 | " | 6-$NO_2$ | $OCH_3$ | Cl | CH | |
| 415 | $CH_2CH{=}CH_2$ | 3,5-$Cl_2$ | $OCH_3$ | $OCH_3$ | CH | |
| 416 | " | 3,5-$Cl_2$ | $OCF_2H$ | $OCF_2H$ | CH | |
| 417 | " | 4,6-$Cl_2$ | $OCH_3$ | $OCH_3$ | CH | |
| 418 | " | 3,5-$Cl_2$ | $OCH_3$ | $CH_3$ | N | |
| 419 | " | 3,5-$F_2$ | $OCH_3$ | $OCH_3$ | CH | |
| 420 | " | 3,5-$F_2$ | $OCH_3$ | Cl | CH | |
| 421 | " | 4,6-$F_2$ | $OCH_3$ | $OCH_3$ | CH | |
| 422 | " | 3,5-$F_2$ | $OCH_2CF_3$ | $OCH_3$ | CH | |
| 423 | $CH_2CH_2OCH_3$ | 3,5-$Cl_2$ | $OCH_3$ | $OCH_3$ | CH | |
| 424 | " | 3,5-$Cl_2$ | $OCH_3$ | $CH_3$ | N | |
| 425 | " | 4,6-$Cl_2$ | $OCH_3$ | $OCH_3$ | CH | |
| 426 | " | 3,5-$Cl_2$ | $OCH_3$ | Cl | CH | |
| 427 | " | 3,5-$F_2$ | $OCH_3$ | $OCH_3$ | CH | |
| 428 | " | 3,5-$F_2$ | $OCH_2CF_3$ | $OCH_3$ | N | |
| 429 | " | 4,6-$F_2$ | $OCH_3$ | $OCH_3$ | CH | |
| 430 | " | 3,5-$F_2$ | $OCH_3$ | $OCH_3$ | N | |

**Tabelle 1b: Fortsetzung**

| Bsp. Nr. | $R^1$ | $(R^2)_n$ | $R^5$ | $R^6$ | E | Fp. |
|---|---|---|---|---|---|---|
| 431 | $CH_2CF_3$ | 3,5-$Cl_2$ | $OCH_3$ | $OCH_3$ | CH | |
| 432 | " | 3,5-$Cl_2$ | $OCH_3$ | $CH_3$ | N | |
| 433 | " | 4,6-$Cl_2$ | $OCH_3$ | $OCH_3$ | CH | |
| 434 | " | 3,5-$Cl_2$ | $OCH_3$ | Cl | CH | |
| 435 | " | 3,5-$F_2$ | $OCH_3$ | $OCH_3$ | CH | |
| 436 | " | 3,5-$F_2$ | $OCH_3$ | $CH_3$ | CH | |
| 437 | " | 4,6-$F_2$ | $OCH_3$ | $OCH_3$ | CH | |
| 438 | " | 3,5-$F_2$ | $OCH_3$ | $CH_3$ | N | |
| 439 | $CH_2CH_2Cl$ | 3,5-$Cl_2$ | $OCH_3$ | $OCH_3$ | CH | |
| 440 | " | 3,5-$Cl_2$ | $OCH_3$ | Cl | CH | |
| 441 | " | 4,6-$Cl_2$ | $OCH_3$ | $OCH_3$ | CH | |
| 442 | " | 3,5-$Cl_2$ | $OCF_2H$ | $CH_3$ | CH | |
| 443 | " | 3,5-$F_2$ | $OCH_3$ | $OCH_3$ | CH | |
| 444 | " | 3,5-$F_2$ | $OCH_3$ | $CH_3$ | N | |
| 445 | " | 4,6-$F_2$ | $OCH_3$ | $OCH_3$ | CH | |
| 446 | " | 3,5-$F_2$ | $OCH_3$ | Cl | CH | |
| 447 | $CH_2CCl=CCl_2$ | 3,5-$Cl_2$ | $OCH_3$ | $OCH_3$ | CH | |
| 448 | " | 3,5-$Cl_2$ | $OCF_2H$ | $OCF_2H$ | CH | |
| 449 | " | 3,5-$F_2$ | $OCH_3$ | $OCH_3$ | CH | |
| 450 | " | 3,5-$F_2$ | $OCH_3$ | $CH_3$ | N | |
| 451 | $CH_2C{\equiv}CCH_3$ | 3,5-$Cl_2$ | $OCH_3$ | $OCH_3$ | CH | |
| 452 | " | 3,5-$Cl_2$ | $OCH_3$ | Cl | CH | |
| 453 | " | 3,5-$F_2$ | $OCH_3$ | $OCH_3$ | CH | |
| 454 | " | 3,5-$F_2$ | $OCH_2CF_3$ | $OCH_3$ | CH | |
| 455 | $CH_2CH=CH_2$ | 3-Cl | $OCH_3$ | $OCH_3$ | CH | 131-133 |
| 456 | " | 3-Cl | $OCH_3$ | $CH_3$ | CH | 77- 82 |
| 457 | " | 3-$C_2H_5$ | $CH_3$ | Cl | CH | Glas |
| 458 | " | 3-$C_2H_5$ | $OC_2H_5$ | $NHCH_3$ | N | 133-135 |
| 459 | " | 3-$C_2H_5$ | $OCH_3$ | $OCH_2CF_3$ | N | 89- 93 |
| 460 | " | 3-$C_2H_5$ | $OCH_3$ | $CH_3$ | N | Glas |
| 461 | " | 3-$C_2H_5$ | $OCH_3$ | $CH_3$ | CH | 136 |
| 462 | " | 3-$C_2H_5$ | $OCH_3$ | $OCH_3$ | CH | 76- 78 |
| 463 | " | 4-F | $OCH_3$ | $CH_3$ | CH | 93- 99 |

**Tabelle 1b: Fortsetzung**

| Bsp. Nr. | $R^1$ | $(R^2)_n$ | $R^5$ | $R^6$ | E | Fp. |
|---|---|---|---|---|---|---|
| 464 | " | 3-F | $OCH_3$ | $CH_3$ | CH | |
| 465 | " | 3-F | $OCH_3$ | $CH_3$ | N | |
| 466 | $CH_2C{\equiv}CH$ | 3-$CH_3$ | $OCH_3$ | $CH_3$ | CH | 167-169 |
| 467 | " | 3-$CH_3$ | $OCH_3$ | $CH_3$ | N | 127-133(Zers.) |
| 468 | " | 3-$OCH_3$ | $OCH_3$ | $OCH_3$ | CH | 186 |
| 469 | " | 3-$OCH_3$ | $OCH_3$ | $CH_3$ | CH | 158-159 |
| 470 | " | 3-$OCH_3$ | $OCH_3$ | $CH_3$ | N | 141-143 |
| 471 | " | 3-Cl | $OCH_3$ | $OCH_3$ | CH | |
| 472 | " | 3-Cl | $OCH_3$ | $CH_3$ | CH | |
| 473 | " | 3-Cl | $OCH_3$ | $CH_3$ | N | |
| 474 | " | H | $CH_3$ | $CH_3$ | CH | 125-126 |
| 475 | " | H | $OC_2H_5$ | $NHCH_3$ | N | 132-134 |
| 476 | " | 3-F | $OCH_3$ | $CH_3$ | CH | |
| 477 | " | 6-F | $OCH_3$ | $OCH_3$ | CH | |

**Tabelle 1c**

| Bsp. Nr. | $R^1$ | $(R^2)_n$ | $R^3$ | $R^5$ | $R^6$ | E | Fp. |
|---|---|---|---|---|---|---|---|
| 478 | $CH_2CH_2Cl$ | H | $CH_3$ | $OCH_3$ | $OCH_3$ | CH | |
| 479 | " | H | $CH_3$ | $OCH_3$ | $CH_3$ | N | |
| 480 | " | H | $CH_2CH=CH_2$ | $OCH_3$ | $OCH_3$ | CH | |
| 481 | " | H | $CH_2CH=CH_2$ | $OCH_3$ | Cl | CH | |
| 482 | $CH_2CH_2OCH_3$ | H | $CH_3$ | $OCH_3$ | $OCH_3$ | CH | |
| 483 | " | H | $CH_3$ | $OCH_3$ | $CH_3$ | CH | |
| 484 | " | H | $CH_2CH=CH_2$ | $OCH_3$ | $OCH_3$ | CH | |
| 485 | " | H | $CH_2CH=CH_2$ | $OCH_3$ | $CH_3$ | N | |
| 486 | $CH_2CH=CH_2$ | H | $CH_3$ | $OCH_3$ | $OCH_3$ | CH | |
| 487 | " | H | $CH_3$ | $OCH_3$ | Cl | CH | |
| 488 | " | H | $CH_2CH=CH_2$ | $OCH_3$ | $OCH_3$ | CH | |
| 489 | " | H | $CH_2CH=CH_2$ | $OCF_2H$ | $CH_3$ | CH | |
| 490 | $CH_2CF_3$ | H | $CH_3$ | $OCH_3$ | $OCH_3$ | CH | |
| 491 | " | H | $CH_3$ | $OCH_3$ | $CH_3$ | N | |
| 492 | " | H | $CH_2CH=CH_2$ | $OCH_3$ | $OCH_3$ | CH | |
| 493 | " | H | $CH_2CH=CH_2$ | $OCH_3$ | Cl | CH | |
| 494 | $CH_2C{\equiv}CH$ | H | $CH_3$ | $OCH_3$ | $OCH_3$ | CH | |
| 495 | " | H | $CH_2CH=CH_2$ | $OCF_2H$ | $OCF_2H$ | CH | |
| 496 | $CH_2CH=CHCl$ | H | $CH_2CH=CH_2$ | $OCH_3$ | $OCH_3$ | CH | |
| 497 | " | H | $CH_3$ | $OCH_3$ | $CH_3$ | N | |
| 498 | $CH_2CH_2Cl$ | H | $C_2H_5$ | $OCH_3$ | $OCH_3$ | CH | |
| 499 | $CH_2CH_2OCH_3$ | H | $C_2H_5$ | $OCH_3$ | Cl | CH | |
| 500 | $CH_2CH=CH_2$ | H | $C_2H_5$ | $OCH_3$ | $OCH_3$ | CH | |
| 501 | $CH_2CF_3$ | H | $C_2H_5$ | $OCH_2CF_3$ | $OCH_3$ | CH | |
| 502 | $CH_2C{\equiv}CH$ | H | $C_2H_5$ | $OCH_3$ | $OCH_3$ | CH | |
| 503 | $CH_2CH_2Cl$ | 3-$CH_3$ | $CH_3$ | $OCH_3$ | $CH_3$ | N | |
| 504 | $CH_2CH_2OCH_3$ | 3-$OCH_3$ | $CH_3$ | $OCH_3$ | $OCH_3$ | CH | |
| 505 | $CH_2CH=CH_2$ | 4-Cl | $CH_2CH=CH_2$ | $OCH_3$ | Cl | CH | |
| 506 | $CH_2CF_3$ | 6-F | $CH_2CH=CH_2$ | $OCH_3$ | $OCH_3$ | CH | |
| 507 | $CH_2C{\equiv}CH$ | 5-$CF_3$ | $CH_3$ | $OCH_2CF_3$ | $OCH_3$ | N | |

Tabelle 2

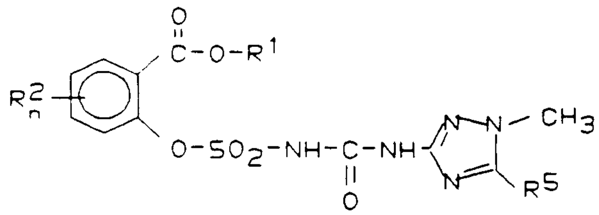

| Bsp. Nr. | $R^1$ | $(R^2)_n$ | $R^5$ | Fp. |
|---|---|---|---|---|
| 508 | $CH_2CH=CH_2$ | H | $CH_3$ | |
| 509 | " | H | H | |
| 510 | " | H | $OCH_3$ | |
| 511 | $CH_2CH_2Cl$ | H | $OCH_3$ | |
| 512 | $CH_2CH_2OCH_3$ | H | $OCH_3$ | |
| 513 | $CH_2C{\equiv}CH$ | H | $OCH_3$ | |
| 514 | $CH_2CF_3$ | H | $OCH_3$ | |
| 515 | $CH_2CH=CHCl$ | H | $OCH_3$ | |
| 516 | $CH_2CCl=CCl_2$ | H | $OCH_3$ | |
| 517 | $CH_2CH_2Br$ | H | $OCH_3$ | |
| 518 | $CH_2CH=CHCH_3$ | H | $OCH_3$ | |
| 519 | $CH_2C{\equiv}CCH_3$ | H | $OCH_3$ | |
| 520 | $CH_2CH=CH_2$ | 3-$CH_3$ | $OCH_3$ | |
| 521 | $CH_2CH_2Cl$ | 3-$OCH_3$ | $OCH_3$ | |
| 522 | $CH_2CH_2OCH_3$ | 4-Cl | $OCH_3$ | |
| 523 | $CH_2CF_3$ | 5-$CF_3$ | $OCH_3$ | |
| 524 | $CH_2C{\equiv}CH$ | 6-F | $OCH_3$ | |
| 525 | $CH_2CH=CHCH_3$ | 4-$OCF_2H$ | $OCH_3$ | |

**Biologische Beispiele**

Die Schädigung der Unkrautpflanzen bzw. die Kulturpflanzenverträglichkeit wurde gemäß einem Schlüssel bonitiert, in dem die Wirksamkeit durch Wertzahlen von 0 - 5 ausgedrückt ist. Dabei bedeutet:

0 = ohne Wirkung
1 = 0 - 20 % Wirkung bzw. Schaden
2 = 20 - 40 % Wirkung bzw. Schaden
3 = 40 - 60 % Wirkung bzw. Schaden
4 = 60 - 80 % Wirkung bzw. Schaden
5 = 80 - 100 % Wirkung bzw. Schaden

### 1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 - 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 - 4 Wochen im Vergleich zu unbehandelten Kontrollen. Wie die Boniturwerte in Tabelle 2 zeigen, weisen die erfindungsgemäßen Verbindungen eine gute herbizide Vorauflaufwirksamkeit gegen ein breites Spektrum von Ungräsern und Unkräutern auf.

### 2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 - 800 l/ha auf die grünen Pflanzenteile gesprüht und nach ca. 3 - 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf (Tabelle 3).

### 3. Kulturpflanzenverträglichkeit

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben, besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z. B. Soja, Baumwolle, Raps, Zuckerrüben und Kartoffeln im Vor- und Nachauflaufverfahren selbst bei hohen Wirkstoffdosierungen ungeschädigt ließen. Einige Substanzen schonten darüber hinaus auch Gramineen-Kulturen wie z. B. Gerste, Weizen, Roggen, Sorghum-Hirsen, Mais oder Reis. Die Verbindungen der Formel I weisen somit eine hohe Selektivität bei Anwendung zur Bekämpfung von unerwünschtem Pflanzenwuchs in landwirtschaftlichen Kulturen auf.

Tabelle 2

| Vorauflaufwirkung der erfindungsgemäßen Verbindungen | | | | | | | |
|---|---|---|---|---|---|---|---|
| Bsp. Nr. | Dosierung (kg/ a.i./ha) | herbizide Wirkung | | | | | |
| | | SIA | CRS | STM | AS | ECG | LOM |
| 4 | 0,6 | 5 | 5 | 5 | 2 | 3 | 4 |
| 3 | 0,6 | 5 | 5 | 5 | 3 | 4 | 3 |
| 74 | 0,6 | 5 | 5 | 2 | 1 | 2 | 1 |
| 5 | 0,6 | 5 | 4 | 2 | 1 | 2 | 1 |
| 10 | 0,6 | 5 | 5 | 3 | 2 | 2 | 2 |
| 6 | 0,6 | 5 | 5 | 5 | 2 | 2 | 2 |
| 138 | 0,6 | 4 | 4 | 4 | 1 | 3 | 3 |
| 139 | 0,6 | 3 | 4 | 4 | 1 | 3 | 3 |

Tabelle 3

| Nachlaufwirkung der erfindungsgemäßen Verbindungen | | | | | | | |
|---|---|---|---|---|---|---|---|
| Bsp. Nr. | Dosierung (kg/ a.i./ha) | herbizide Wirkung | | | | | |
| | | SIA | CRS | STM | AS | ECG | LOM |
| 4 | 0,6 | 5 | 5 | 5 | 1 | 3 | 4 |
| 3 | 0,6 | 5 | 5 | 5 | 1 | 2 | 3 |
| 74 | 0,6 | 4 | 2 | 1 | 1 | 1 | 1 |
| 5 | 0,6 | 4 | 3 | 1 | 1 | 2 | 1 |
| 10 | 0,6 | 5 | 5 | 4 | 1 | 0 | 1 |
| 6 | 0,6 | 5 | 5 | 5 | 0 | 3 | 4 |
| 138 | 0,6 | 5 | 5 | 5 | 1 | 4 | 4 |
| 139 | 0,6 | 5 | 5 | 5 | 1 | 3 | 3 |

Abkürzungen:
SIA = Sinapis alba
CRS = Chrysanthemum segetum
STM = Stellaria media
AS = Avena sativa
ECG = Echinochloa crus-galli
LOM = Lolium multiflorum
a.i. = Aktivsubstanz

**Patentansprüche**
**Patentansprüche für folgende Vertragsstaaten : BE, CH, DE, FR, GB, IT, LI, NL**

**1.** Verbindungen der Formel I oder deren Salze

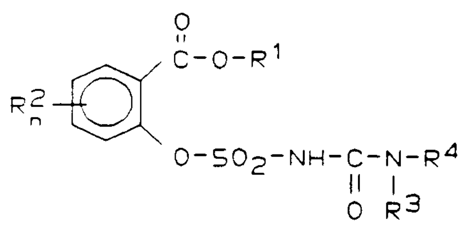

worin

$R^1$ $(C_1-C_8)$-Alkyl, das ein- oder mehrfach durch Halogen oder ein- bis zweifach durch $(C_1-C_4)$-Alkoxy substituiert ist; $(C_2-C_8)$-Alkenyl, $(C_2-C_8)$-Alkinyl, die beide gegebenenfalls ein- oder mehrfach durch Halogen oder ein- bis zweifach durch $(C_1-C_4)$-Alkoxy substituiert sein können,

$R^2$ unabhängig voneinander Halogen; Nitro; $(C_1-C_4)$-Alkyl oder $(C_1-C_4)$-Alkoxy, die beide gegebenenfalls ein- oder mehrfach durch Halogen substituiert sein können,

$R^3$ Wasserstoff; $(C_1-C_8)$-Alkyl; $(C_2-C_8)$-Alkenyl oder $(C_2-C_8)$-Alkinyl,

$R^4$ einen heterocyclischen Rest der Formeln

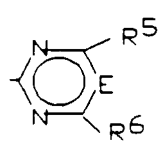 oder 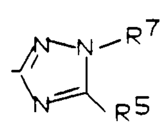 ,

wobei E = CH oder N ist,

n 0, 1, 2 oder 3,

$R^5$,$R^6$ unabhängig voneinander Wasserstoff; Halogen; ($C_1$-$C_4$)-Alkyl oder ($C_1$-$C_4$)-Alkoxy, die beide gegebenenfalls ein- oder mehrfach halogeniert sein können; Di-($C_1$-$C_4$)-alkoxy-($C_1$-$C_2$)-alkyl; Cyclopropyl, -$OCHR^8COOR^9$; -$NR^9R^{10}$ oder ($C_1$-$C_4$)-Alkylthio,

$R^7$ ($C_1$-$C_4$)-Alkyl,

$R^8$ Wasserstoff oder ($C_1$-$C_4$)-Alkyl und

$R^9$,$R^{10}$ unabhängig voneinander Wasserstoff; ($C_1$-$C_4$)-Alkyl; ($C_2$-$C_4$)-Alkenyl oder ($C_2$-$C_4$)-Alkinyl

bedeuten.

**2.** Verbindungen der Formel I von Anspruch 1 und deren Salze, wobei bei Formel I $R^1$ ($C_1$-$C_4$)Alkyl, das wie in Anspruch 1 beschrieben substituiert ist, ($C_3$-$C_4$)-Alkenyl, das wie in Anspruch 1 beschrieben substituiert sein kann, oder Propargyl; $R^2$ Halogen, ($C_1$-$C_3$)-Alkyl oder ($C_1$-$C_3$)-Alkoxy, die wie in Anspruch 1 beschrieben substituiert sein können; n = 0, 1 oder 2; $R^3$ Wasserstoff, ($C_1$-$C_4$)-Alkyl oder Allyl; $R^4$ einen Rest der Formel

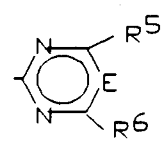

und $R^5$ und $R^6$ unabhängig voneinander Halogen, ($C_1$-$C_4$)-Alkyl oder ($C_1$-$C_4$)-Alkoxy, die beide durch Halogen substituiert sein können, bedeuten.

**3.** Verbindungen der Formel I von Ansprüchen 1 und 2 und deren Salze, wobei $R^1$ ($C_1$-$C_4$)-Alkyl, das wie in Anspruch 1 beschrieben substituiert ist, ($C_3$-$C_4$)-Alkenyl, das wie in Anspruch 1 beschrieben substituiert sein kann, oder Propargyl; n = 0; $R^3$ Wasserstoff, $R^4$ einen Rest der Formel

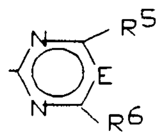

und $R^5$ und $R^6$ unabhängig voneinander Chlor, Brom, ($C_1$-$C_4$)-Alkyl, ($C_1$-$C_4$)-Alkoxy, $OCF_2H$, $OCH_2CF_3$ oder $CF_3$ bedeuten.

**4.** Verfahren zur Herstellung der Verbindungen der Formel I gemäß Ansprüchen 1 bis 3, dadurch gekennzeichnet, daß man

(a) eine Verbindung der Formel (II)

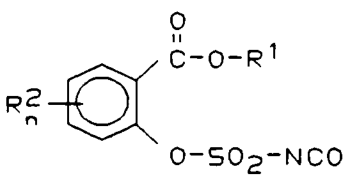

(II)

mit einer Verbindung der Formel (III)

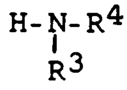

(III)

umsetzt, oder

(b) eine Verbindung der Formel (IV)

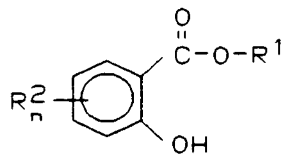

mit einem Chlorsulfonylharnstoff der Formel (V)

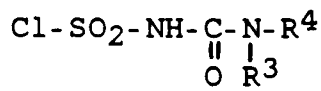

(V)

umsetzt, oder

(c) eine substituierte Benzoesäure der Formel (VI)

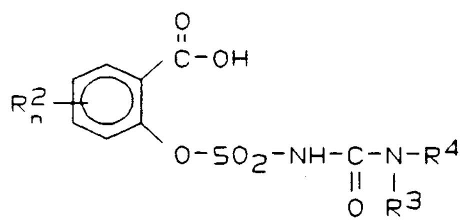

(VI)

mit einem Alkylierungsreagens der Formel (VII)

$R^1$-X (VII),

wobei X für eine nucleofuge Abgangsgruppe wie z. B. Halogen, Alkyl-$SO_2$-O- oder Tosyl steht, umsetzt.

**5.** Herbizide Mittel, dadurch gekennzeichnet, daß sie eine Verbindung der Formel I von Ansprüchen 1 bis 3 oder deren Salze neben inerten Trägerstoffen enthalten.

**6.** Pflanzenwachstumsregulierende Mittel, dadurch gekennzeichnet, daß sie eine Verbindung der Formel I von Ansprüchen 1 bis 3 oder deren Salze neben inerten Trägerstoffen enthalten.

**7.** Verwendung von Verbindungen der Formel I oder deren Salze gemäß Ansprüchen 1 bis 3 als Herbizide oder Pflanzenwachstumsregulatoren.

**8.** Verfahren zur Bekämpfung von unerwünschten Pflanzen, dadurch gekennzeichnet, daß man auf diese oder deren Anbauflächen eine wirksame Menge einer Verbindung der Formel I oder deren Salze von Ansprüchen 1 bis 3 appliziert.

**9.** Verfahren zur Wachstumsregulierung von Pflanzen, dadurch gekennzeichnet, daß man auf diese oder die Anbauflächen eine wirksame Menge einer Verbindung der Formel I oder deren Salze von Ansprüchen 1 bis 3 appliziert.

**Patentansprüche für folgenden Vertragsstaat : ES**

**1.** Verfahren zur Herstellung der Verbindungen der Formel I oder deren Salze

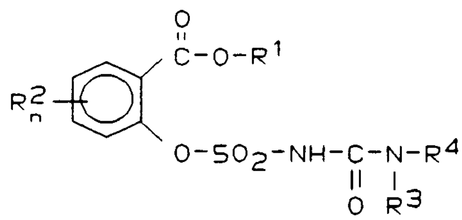

(I)

worin

$R^1$ $(C_1\text{-}C_8)$-Alkyl, das ein- oder mehrfach durch Halogen oder ein- bis zweifach durch $(C_1\text{-}C_4)$-Alkoxy substituiert ist; $(C_2\text{-}C_8)$-Alkenyl, $(C_2\text{-}C_8)$ Alkinyl, die beide gegebenenfalls ein- oder mehrfach durch Halogen oder ein- bis zweifach durch $(C_1\text{-}C_4)$-Alkoxy substituiert sein können,

$R^2$ unabhängig voneinander Halogen; Nitro; $(C_1\text{-}C_4)$-Alkyl oder $(C_1\text{-}C_4)$-Alkoxy, die beide gegebenenfalls ein- oder mehrfach durch Halogen substituiert sein können,

$R^3$ Wasserstoff; $(C_1\text{-}C_8)$-Alkyl; $(C_2\text{-}C_8)$-Alkenyl oder $(C_2\text{-}C_8)$-Alkinyl,

$R^4$ einen heterocyclischen Rest der Formeln

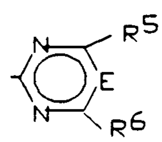

oder

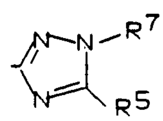

,

wobei E = CH oder N ist,

n 0, 1, 2 oder 3,

$R^5$,$R^6$ unabhängig voneinander Wasserstoff; Halogen; $(C_1\text{-}C_4)$-Alkyl oder $(C_1\text{-}C_4)$-Alkoxy, die beide gegebenenfalls ein- oder mehrfach halogeniert sein können; Di-$(C_1\text{-}C_4)$-alkoxy-$(C_1\text{-}C_2)$-alkyl; Cyclopropyl, $\text{-OCHR}^8\text{COOR}^9$; $\text{-NR}^9\text{R}^{10}$ oder $(C_1\text{-}C_4)$-Alkylthio,

$R^7$ $(C_1\text{-}C_4)$-Alkyl,

$R^8$ Wasserstoff oder $(C_1\text{-}C_4)$-Alkyl und

$R^9$,$R^{10}$ unabhängig voneinander Wasserstoff; $(C_1\text{-}C_4)$-Alkyl; $(C_2\text{-}C_4)$-Alkenyl oder $(C_2\text{-}C_4)$-Alkinyl

bedeuten, dadurch gekennzeichnet, daß man

(a) eine Verbindung der Formel (II)

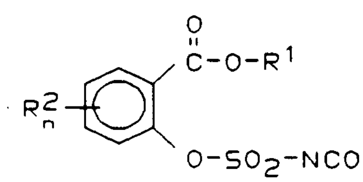

(II)

mit einer Verbindung der Formel (III)

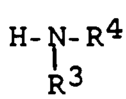

(III)

umsetzt, oder

(b) eine Verbindung der Formel (IV)

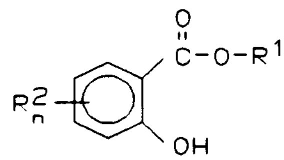

mit einem Chlorsulfonylharnstoff der Formel (V)

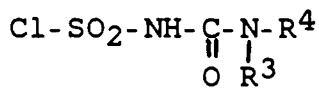

(V)

umsetzt, oder

(c) eine substituierte Benzoesäure der Formel (VI)

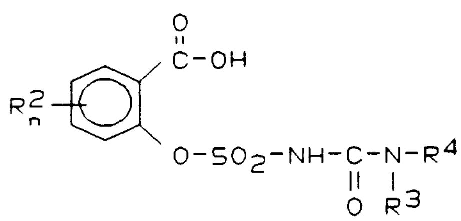

(VI)

mit einem Alkylierungsreagens der Formel (VII)

$R^1$-X (VII),

wobei X für eine nucleofuge Abgangsgruppe wie z. B. Halogen, Alkyl-$SO_2$-O- oder Tosyl steht, umsetzt.

**2.** Verfahren gemäß Anspruch 1, dadurch gekennzeichnet, daß $R^1$ ($C_1$-$C_4$)Alkyl, das wie in Anspruch 1 beschrieben substituiert ist, ($C_3$-$C_4$)-Alkenyl, das wie in Anspruch 1 beschrieben substituiert sein kann oder Propargyl; $R^2$ Halogen, ($C_1$-$C_3$)-Alkyl oder ($C_1$-$C_3$)-Alkoxy, die wie in Anspruch 1 beschrieben substituiert sein können; n = 0, 1 oder 2; $R^3$ Wasserstoff, ($C_1$-$C_4$)-Alkyl oder Allyl; $R^4$ einen Rest der Formel

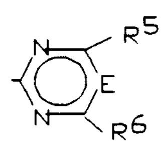

und $R^5$ und $R^6$ unabhängig voneinander Halogen, ($C_1$-$C_4$)-Alkyl oder ($C_1$-$C_4$)-Alkoxy, die beide durch Halogen substituiert sein können, bedeuten.

**3.** Verfahren gemäß Ansprüchen 1 oder 2, dadurch gekennzeichnet, daß $R^1$ ($C_1$-$C_4$)-Alkyl, das wie in Anspruch 1 beschrieben substituiert ist, ($C_3$-$C_4$)-Alkenyl, das wie in Anspruch 1 beschrieben substituiert sein kann oder Propargyl; n = 0; $R^3$ Wasserstoff, $R^4$ einen Rest der Formel

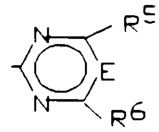

und $R^5$ und $R^6$ unabhängig voneinander Chlor, Brom, $(C_1-C_4)$-Alkyl, $(C_1-C_4)$-Alkoxy, $OCF_2H$, $OCH_2CF_3$ oder $CF_3$ bedeuten.

**4.** Verfahren zur Bekämpfung von unerwünschten Pflanzen, dadurch gekennzeichnet, daß man auf diese oder deren Anbauflächen eine wirksame Menge einer Verbindung der Formel I oder deren Salze von Ansprüchen 1 bis 3 appliziert.

**5.** Verfahren zur Wachstumsregulierung von Pflanzen, dadurch gekennzeichnet, daß man auf diese oder die Anbauflächen eine wirksame Menge einer Verbindung der Formel I oder deren Salze von Ansprüchen 1 bis 3 appliziert.

**Claims**

**Claims for the following Contracting States : BE, CH, DE, FR, GB, IT, LI, NL**

**1.** Compounds of the formula I or their salts

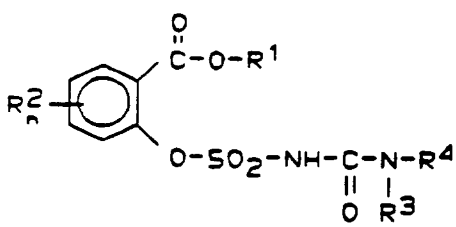

wherein

| | |
|---|---|
| $R^1$ | denotes $(C_1-C_8)$-alkyl which is monosubstituted or polysubstituted by halogen or monosubstituted or disubstituted by $(C_1-C_4)$-alkoxy; $(C_2-C_8)$-alkenyl and $(C_2-C_8)$-alkynyl, both of which can be unsubstituted or monosubstituted or polysubstituted by halogen or monosubstituted or disubstituted by $(C_1-C_4)$-alkoxy, |
| $R^2$ | denotes, independently of the other $R^2$s, halogen; nitro; $(C_1-C_4)$-alkyl or $(C_1-C_4)$-alkoxy, both of which can be unsubstituted or monosubstituted or polysubstituted by halogen, |
| $R^3$ | denotes hydrogen; $(C_1-C_8)$-alkyl; $(C_2-C_8)$-alkenyl or $(C_2-C_8)$-alkynyl, |
| $R^4$ | denotes a heterocyclic radical of the formula |

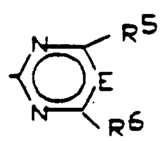

or

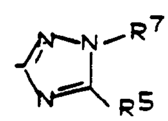

| | |
|---|---|
| | wherein E is CH or N, |
| n | denotes 0, 1, 2 or 3, |
| $R^5$ and $R^6$, | independently of one another, denote hydrogen; halogen; $(C_1-C_4)$-alkyl or $(C_1-C_4)$-alkoxy, both of which can be unsubstituted or monosubstituted or polysubstituted by halogen; di-$(C_1-C_4)$-alkoxy-$(C_1-C_2)$-alkyl; cyclopropyl, $-OCHR^8COOR^9$; $-NR^9R^{10}$ or $(C_1-C_4)$-alkylthio, |
| $R^7$ | denotes $(C_1-C_4)$-alkyl, |
| $R^8$ | denotes hydrogen or $(C_1-C_4)$-alkyl and |
| $R^9$ and $R^{10}$, | independently of one another, denote hydrogen; $(C_1-C_4)$-alkyl; $(C_2-C_4)$-alkenyl or |

$(C_2-C_4)$-alkynyl.

**2.** Compounds of the formula I of claim 1 and their salts, in which, in formula I, $R^1$ denotes $(C_1-C_4)$-alkyl which is substituted as described in claim 1, $(C_3-C_4)$-alkenyl which can be substituted as described in claim 1, or propargyl; $R^2$ denotes halogen, $(C_1-C_3)$-alkyl or $(C_1-C_3)$-alkoxy, which can be substituted as described in claim 1; n denotes 0, 1 or 2; $R^3$ denotes hydrogen, $(C_1-C_4)$-alkyl or allyl; $R^4$ denotes a radical of the formula

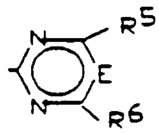

and $R^5$ and $R^6$, independently of one another, denote halogen, $(C_1-C_4)$-alkyl or $C_1-C_4)$-alkoxy, both of which can be substituted by halogen.

**3.** Compounds of the formula I of claims 1 and 2 and their salts, in which $R^1$ denotes $(C_1-C_4)$-alkyl which is substituted as described in claim 1, or $(C_3-C_4)$-alkenyl which can be substituted as described in claim 1, or propargyl; n denotes 0; $R^3$ denotes hydrogen, $R^4$ denotes a radical of the formula

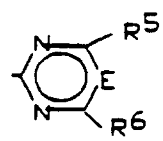

and $R^5$ and $R^6$, independently of one another, denote chlorine, bromine, $(C_1-C_4)$-alkyl, $(C_1-C_4)$-alkoxy, $OCF_2H$, $OCH_2CF_3$ or $CF_3$.

**4.** A process for the preparation of compounds of the formula I as claimed in claims 1 to 3, wherein
(a) a compound of the formula (II)

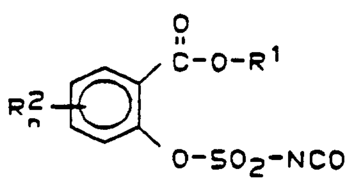

(II)

is reacted with a compound of the formula (III)

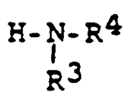

(III)

or
(b) a compound of the formula (IV)

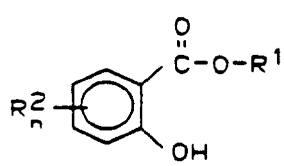

is reacted with a chlorosulfonylurea of the formula (V)

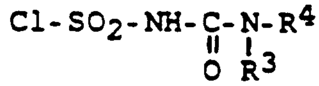

(V)

or

(c) a substituted benzoic acid of the formula (VI)

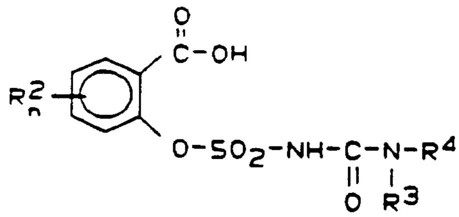

(VI)

is reacted with an alkylating reagent of the formula (VII)

$R^1$-X (VII)

where X represents a nucleofugic leaving group, such as, for example, halogen, alkyl-$SO_2$-O- or tosyl.

**5.** A herbicidal composition, which contains a compound of the formula I of claims 1 to 3 or salts thereof, in addition to inert carriers.

**6.** A plant growth-regulating composition, which contains a compound of the formula I of claims 1 to 3 or salts thereof, in addition to inert carriers.

**7.** The use of compounds of the formula I or their salts as claimed in claims 1 to 3 as herbicides or plant growth-regulators.

**8.** A method of controlling unwanted plants, which comprises applying to these plants or the areas where they grow an effective quantity of a compound of the formula I or salts thereof of claims 1 to 3.

**9.** A method of regulating the growth of plants, which comprises applying to these plants or the areas where they grow an effective quantity of a compound of the formula I or salts thereof of claims 1 to 3.

**Claims for the following Contracting State : ES**

**1.** A process for the preparation of compounds of the formula I or their salts

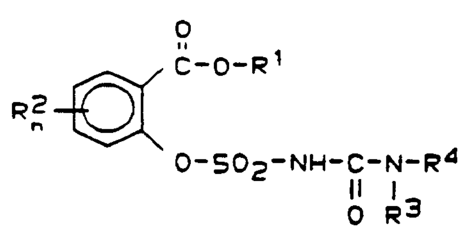

(I)

wherein

$R^1$ denotes ($C_1$-$C_8$)-alkyl which is monosubstituted or polysubstituted by halogen or monosubstituted or disubstituted by ($C_1$-$C_4$)-alkoxy; ($C_2$-$C_8$)-alkenyl and ($C_2$-$C_8$)-alkynyl, both of which can be unsubstituted or monosubstituted or polysubstituted

by halogen or monosubstituted or disubstituted by ($C_1$-$C_4$)-alkoxy,

$R^2$ denotes, independently of the other $R^2$s, halogen; nitro; ($C_1$-$C_4$)-alkyl or ($C_1$-$C_4$)-alkoxy, both of which can be unsubstituted or monosubstituted or polysubstituted by halogen,

$R^3$ denotes hydrogen; ($C_1$-$C_8$)-alkyl; ($C_2$-$C_8$)-alkenyl or ($C_2$-$C_8$)-alkynyl,

$R^4$ denotes a heterocyclic radical of the formula

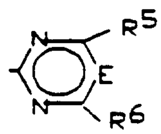

or

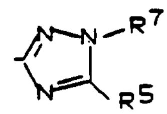

wherein E is CH or N,

n denotes 0, 1, 2 or 3,

$R^5$ and $R^6$, independently of one another, denote hydrogen; halogen; ($C_1$-$C_4$)-alkyl or ($C_1$-$C_4$)-alkoxy, both of which can be unsubstituted or monosubstituted or polysubstituted by halogen; di-($C_1$-$C_4$)-alkoxy-($C_1$-$C_2$)-alkyl; cyclopropyl, $-OCHR^8COOR^9$; $-NR^9R^{10}$ or ($C_1$-$C_4$)-alkylthio,

$R^7$ denotes ($C_1$-$C_4$)-alkyl,

$R^8$ denotes hydrogen or ($C_1$-$C_4$)-alkyl and

$R^9$ and $R^{10}$, independently of one another, denote hydrogen; ($C_1$-$C_4$)-alkyl; ($C_2$-$C_4$)-alkenyl or ($C_2$-$C_4$)-alkynyl.

wherein

(a) a compound of the formula (II)

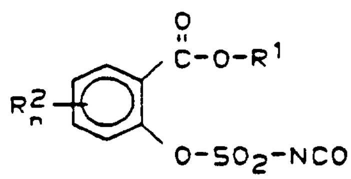

(II)

is reacted with a compound of the formula (III)

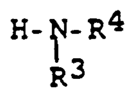

(III)

or

(b) a compound of the formula (IV)

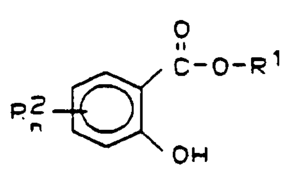

is reacted with a chlorosulfonylurea of the formula (V)

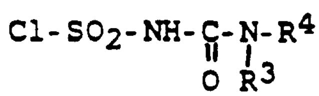

(V)

or

(c) a substituted benzoic acid of the formula (VI)

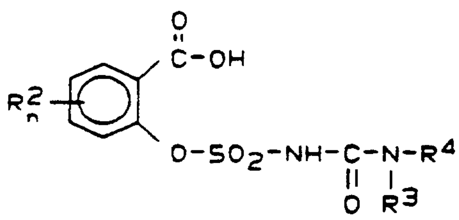

(VI)

is reacted with an alkylating reagent of the formula (VII)

$R^1$-X (VII)

where X represents a nucleofugic leaving group, such as, for example, halogen, alkyl-$SO_2$-O- or tosyl.

**2.** The process as claimed in claim 1, wherein $R^1$ denotes ($C_1$-$C_4$)-alkyl which is substituted as described in claim 1, ($C_3$-$C_4$)-alkenyl which can be substituted as described in claim 1, or propargyl; $R^2$ denotes halogen, ($C_1$-$C_3$)-alkyl or ($C_1$-$C_3$)-alkoxy, which can be substituted as described in claim 1; n denotes 0, 1 or 2; $R^3$ denotes hydrogen, ($C_1$-$C_4$)-alkyl or allyl; $R^4$ denotes a radical of the formula

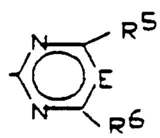

and $R^5$ and $R^6$, independently of one another, denote halogen, ($C_1$-$C_4$)-alkyl or ($C_1$-$C_4$)-alkoxy, both of which can be substituted by halogen.

**3.** The process as claimed in claim 1 or 2, wherein $R^1$ denotes ($C_1$-$C_4$)-alkyl which is substituted as described in claim 1, or ($C_3$-$C_4$)-alkenyl which can be substituted as described in claim 1, or propargyl; n denotes 0; $R^3$ denotes hydrogen, $R^4$ denotes a radical of the formula

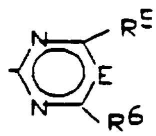

and $R^5$ and $R^6$, independently of one another, denote chlorine, bromine, ($C_1$-$C_4$)-alkyl, ($C_1$-$C_4$)-alkoxy, $OCF_2H$, $OCH_2CF_3$ or $CF_3$.

**4.** A method of controlling unwanted plants, which comprises applying to these plants or the areas where they grow an effective quantity of a compound of the formula I or salts thereof of claims 1 to 3.

**5.** A method of regulating the growth of plants, which comprises applying to these plants or the areas where they grow an effective quantity of a compound of the formula I or salts thereof of claims 1 to 3.

**Revendications**

**Revendications pour les Etats contractants suivants : BE, CH, DE, FR, GB, IT, LI, NL**

**1.** Composés de formule I ou leurs sels

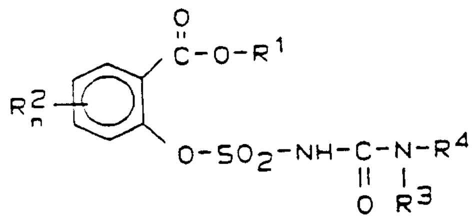

où :

le radical $R^1$ représente un alkyle en $C_1$-$C_8$ substitué une ou plusieurs fois par des halogène ou une ou deux fois par des alcoxy en $C_1$-$C_4$ ; alcényle en $C_2$-$C_8$, alcynyle en $C_2$-$C_8$, les deux radicaux pouvant être éventuellement substitués une ou plusieurs fois par des halogène ou une ou deux fois par des alcoxy en $C_1$-$C_4$,

les radicaux $R^2$ représentent, indépendamment les uns des autres, les halogène ; nitro ; alkyle en $C_1$-$C_4$ ou alcoxy en $C_1$-$C_4$, les deux radicaux pouvant être éventuellement substitués une ou plusieurs fois par un halogène,

le radical $R^3$ représente l'hydrogène ; les alkyle en $C_1$-$C_8$ ; alcényle en $C_2$-$C_8$ ou alcynyle en $C_2$-$C_8$,

le radical $R^4$ représente un radical hétérocyclique de formules

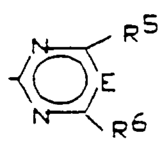

ou

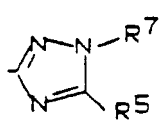

,

où E = CH ou N,

n vaut 0, 1, 2 ou 3,

les radicaux $R^5$, $R^6$, indépendamment l'un de l'autre, représentant l'hydrogène ; les halogène ; alkyle en $C_1$-$C_4$ ou alcoxy en $C_1$-$C_4$, les deux radicaux pouvant être éventuellement halogénés une ou plusieurs fois ; di alcoxy en $C_1$-$C_4$)-(alkyle en $C_1$-$C_2$) ; cyclopropyle, $-OCHR^8COOR^9$ ; $-NR^9R^{10}$ ou alkylthio en $C_1$-$C_4$,

le radical $R^7$ représentant un alkyle en $C_1$-$C_4$,

le radical $R^8$ représentant l'hydrogène ou un alkyle en $C_1$-$C_4$, et

les radicaux $R^9$, $R^{10}$, indépendamment l'un de l'autre, représentant l'hydrogène ; les alkyle en $C_1$-$C_4$ ; alcényle en $C_2$-$C_4$ ou alcynyle en $C_2$-$C_4$.

**2.** Composés de formule I selon la revendication 1 et leurs sels, dans la formule I $R^1$ étant un alkyle en $C_1$-$C_4$ qui est substitué selon la description de la revendication 1, un alcényle en $C_3$-$C_4$ pouvant être substitué selon la description de la revendication 1 ou le propargyle ; $R^2$ représentant les halogène, alkyle en $C_1$-$C_3$ ou alcoxy en $C_1$-$C_3$ pouvant être substitués selon la description de la revendication 1; n = 0, 1 ou 2 ; $R^3$ représentant l'hydrogène, un alkyle en $C_1$-$C_4$ ou un allyle ; $R^4$ représentant un radical de formule

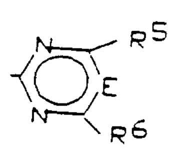

et $R^5$ et $R^6$, les deux radicaux indépendamment l'un de l'autre, représentant les halogène, alkyle en $C_1$-$C_4$ ou alcoxy en $C_1$-$C_4$, les deux radicaux pouvant être substitués par des halogènes.

**3.** Composés de formule I selon les revendications 1 et 2 et leurs sels, $R^1$ étant un alkyle en $C_1$-$C_4$ substitué selon la description de la revendication 1, un alcényle en $C_3$-$C_4$ pouvant être substitué selon la description de la revendication 1 ou le propargyle ; n = 0 ; $R^3$ représentant l'hydrogène, $R^4$ un radical de formule

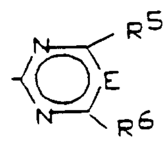

et $R^5$ et $R^6$, indépendamment l'un de l'autre, représentant les chlore, brome, alkyle en $C_1$-$C_4$, alcoxy en $C_1$-$C_4$, $OCF_2H$, $OCH_2CF_3$ ou $CF_3$.

**4.** Procédé pour la préparation des composés de formule I selon les revendications 1 à 3, caractérisé en ce qu'on fait réagir

(a) un composé de formule II

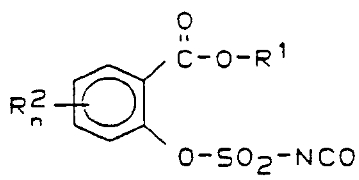

(II)

avec un composé de formule III

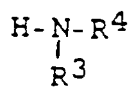

(III)

ou

(b) on fait réagir un composé de formule IV

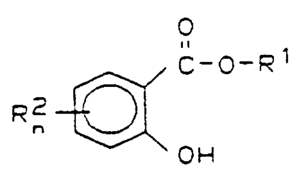

avec une chlorosulfonyle-urée de formule V

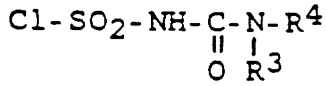

(V)

ou

(c) on fait réagir un acide benzoïque substitué de formule VI

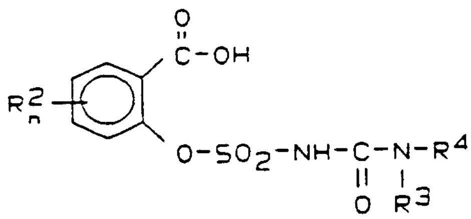

(VI)

avec un réactif d'alkylation de formule VII

$R^1$-X (VII),

X représentant un groupe éliminable nucléofuge, comme par exemple les halogène, alkyle-$SO_2$-O- ou tosyle.

**5.** Agents herbicides caractérisés en ce qu'ils contiennent, outre les substances véhicules inertes, un composé de formule I des revendications 1 à 3 ou ses sels.

**6.** Agents régulateurs de la croissance des plantes, caractérisés en ce qu'ils contiennent un composé de formule I des revendications 1 à 3 ou ses sels, outre les substances véhicules inertes.

**7.** Utilisation des composés de formule I ou de leurs sels selon les revendications 1 à 3, en tant qu'herbicides ou régulateurs de la croissance des plantes.

**8.** Procédé pour la lutte contre les plantes indésirables, caractérisé en ce qu'on applique sur celles-ci ou sur les surfaces cultivables une quantité efficace d'un composé de formule I ou de ses sels selon les revendications 1 à 3.

**9.** Procédé pour la régulation de la croissance des plantes, caractérisé en ce qu'on applique sur celles-ci ou sur les surfaces cultivables une quantité efficace d'un composé de formule I ou de ses sels selon les revendications 1 à 3.

**Revendications pour l'Etat contractant suivant : ES**

**1.** Procédé pour la préparation de composés de formule I ou de leurs sels

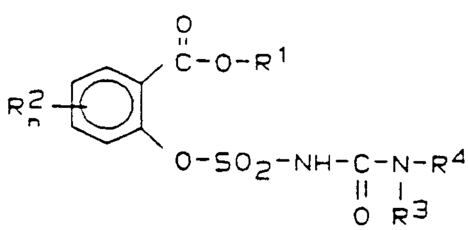

(I)

où

le radical $R^1$ représente un alkyle en $C_1$-$C_8$ substitué une ou plusieurs fois par des halogène ou une ou deux fois par des alcoxy en $C_1$-$C_4$ ; alcényle en $C_2$-$C_8$, alcynyle en $C_2$-$C_8$, les deux radicaux pouvant être éventuellement substitués une ou plusieurs fois par des halogène ou une ou deux fois par des alcoxy en $C_1$-$C_4$,

les radicaux $R^2$ représentent, indépendamment les uns des autres, les halogène ; nitro ; alkyle en $C_1$-$C_4$ ou alcoxy en $C_1$-$C_4$, les deux radicaux pouvant être éventuellement substitués une ou plusieurs fois par un halogène,

le radical $R^3$ représente l'hydrogène ; les alkyle en $C_1$-$C_8$ ; alcényle en $C_2$-$C_8$ ou alcynyle en $C_2$-$C_8$,

le radical $R^4$ représente un radical hétérocyclique de formules

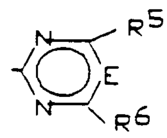

ou

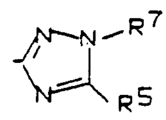

,

où E = CH ou N,

n vaut 0, 1, 2 ou 3,

les radicaux $R^5$, $R^6$, indépendamment l'un de l'autre, représentant l'hydrogène ; les halogène ; alkyle en $C_1$-$C_4$ ou alcoxy en $C_1$-$C_4$, les deux radicaux pouvant être éventuellement halogénés une ou plusieurs fois ; di(alcoxy en $C_1$-$C_4$)-(alkyle en $C_1$-$C_2$) ; cyclopropyle, $-OCHR^8COOR^9$ ; $-NR^9R^{10}$ ou alkylthio en $C_1$-$C_4$,

le radical $R^7$ représentant un alkyle en $C_1$-$C_4$,

le radical $R^8$ représentant l'hydrogène ou un alkyle en $C_1$-$C_4$, et

les radicaux $R^9$, $R^{10}$ indépendamment l'un de l'autre, représentent l'hydrogène ; les alkyle en $C_1$-$C_4$ ; alcényle en $C_2$-$C_4$ ou alcynyle en $C_2$-$C_4$,

caractérisés en ce qu'on fait réagir

(a) un composé de formule II

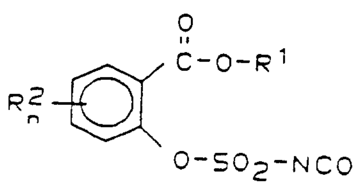

(II)

avec un composé de formule III

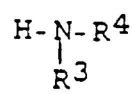

(III)

ou

(b) on fait réagir un composé de formule IV

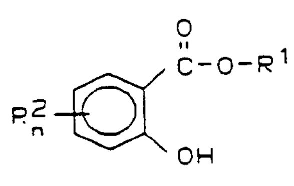

avec une chlorosulfonyle-urée de formule V

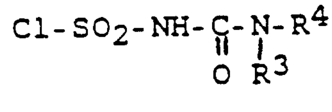

(V)

ou

(c) on fait réagir un acide benzoïque substitué de formule VI

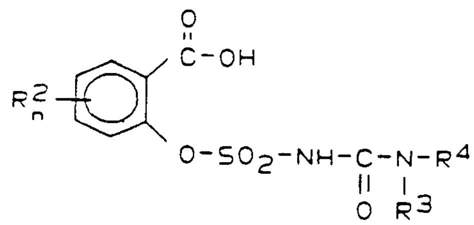

(VI)

avec un réactif d'alkylation de formule VII

$R^1$-X (VII),

X représentant un groupe éliminable nucléofuge, comme par exemple les halogène, alkyle-$SO_2$-O- ou tosyle.

2. Procédé selon la revendication 1, caractérisé en ce que $R^1$ représente un alkyle en $C_1$-$C_4$ substitué selon la description dans la revendication 1, un alcényle en $C_3$-$C_4$ qui peut être substitué selon la description dans la revendication 1 ou le propargyle ; $R^2$ représente les halogène, alkyle en $C_1$-$C_3$ ou alcoxy $C_1$-$C_3$, qui pouvent être substitués selon la description dans la revendication 1; n = 0, 1 ou 2 ; $R^3$ représente l'hydrogène, un alkyle en $C_1$-$C_4$ ou un allyle ; $R^4$ un radical de formule

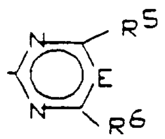

et $R^5$ et $R^6$, les deux radicaux indépendamment l'un de l'autre, représentent les halogène, alkyle en $C_1$-$C_4$ ou alcoxy en $C_1$-$C_4$, les deux radicaux peuvent être substitués par un halogène.

3. Procédé selon les revendications 1 et 2, caractérisé en ce que $R^1$ représente un alkyle en $C_1$-$C_4$ qui est substitué selon la description dans la revendication 1, un alcényle en $C_3$-$C_4$ qui peut être substitué selon la description dans la revendication 1 ou le propargyle ; n = 0 ; $R^3$ représente l'hydrogène, $R^4$ un radical de formule

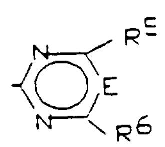

et $R^5$ et $R^6$, indépendamment l'un de l'autre, représentent les chlore, brome, alkyle en $C_1$-$C_4$, alcoxy en $C_1$-$C_4$, $OCF_2H$, $OCH_2CF_3$ ou $CF_3$.

4. Procédé pour la lutte contre les plantes indésirables, caractérisé en ce qu'on applique sur celles-ci ou sur les surfaces cultivables une quantité efficace d'un composé de formule I ou de ses sels des revendications 1 à 3.

5. Procédé pour la régulation de la croissance des plantes, caractérisé en ce qu'on applique sur celles-ci ou sur les surfaces cultivables une quantité efficace d'un composé de formule I ou de ses sels des revendications 1 à 3.